# EUROPEAN PATENT SPECIFICATION

(11) **EP 0 785 950 B1**
(45) Date of publication and mention of the grant of the patent: **16.04.2003**
(21) Application number: 95938216.9
(22) Date of filing: 12.10.1995
(51) Int. Cl.: C07K 14/50, C12N 15/12, A61K 38/18

(54) **KERATINOCYTE GROWTH FACTOR ANALOGS**
ANALOGEN DES KERATINOZYTENWACHSTUMFAKTORS
ANALOGUES DU FACTEUR DE CROISSANCE DES KERATINOCYTES

(30) Priority: 13.10.1994 US 323337; 07.06.1995 US 487825
(43) Date of publication of application: 30.07.1997
(73) Proprietor: Amgen Inc.,, Thousand Oaks, California 91320-1799 (US)
(72) Inventor: CHEN, Bao-Lu, Emeryville, CA 94608 (US); ARAKAWA, Tsutomu, Thousand Oaks, CA 91320 (US)
(74) Representative: Grünecker, Kinkeldey, Stockmair & Schwanhäusser Anwaltssozietät
(86) International application number: US9513075
(87) International publication number: WO96011951

(56) References cited:
- WO-A-90/08771
- WO-A-95/08630
- IN VITRO CELL. DEV. BIOL., vol. 27a, no. 6, June 1991, pages 437-438, XP002016903 G.YAN ET AL: "Sequence of rat KGF"
- BIOCHEMICAL AND BIOPHYSICAL RESEARCH COMMUNICATIONS, vol. 185, no. 3, 30 June 1992, ORLANDO, FL US, pages 1098-1107, XP002016904 M.PRESTA ET AL: "Structure-function relationship of bFGF..."

## Description

### Field of the Invention

The present invention relates to recombinant DNA technology and protein engineering. Specifically, recombinant DNA methodologies have been applied to generate polypeptide analogs of keratinocyte growth factor (KGF), a potent mitogen of non-fibroblast epithelial cell growth, wherein the analogs have improved stability as compared to that of the parent KGF.

### Background

The complex process of tissue generation and regeneration is mediated by a number of protein factors sometimes referred to as soft tissue growth factors. These molecules are generally released by one cell type and act to influence proliferation of other cell types. (Rubin *et al.* (1989), *Proc. Nat'l. Acad. Sci. USA,* 86:802-806). Some soft tissue growth factors are secreted by particular cell types and influence the proliferation, differentiation and/or maturation of responsive cells in the development of multicellular organisms (Finch *et al.* (1989), *Science,* 245:752-755). In addition to their roles in developing organisms, some are significant in the continued health and maintenance of more mature systems. For instance, in mammals there are many systems where rapid cell turnover occurs. Such systems include the skin and the gastrointestinal tract, both of which are comprised of epithelial cells. Included within this group of soft tissue growth factors is a protein family of fibroblast growth factors (FGFs).

There are currently eight known FGF family members which share a relatedness among primary structures: basic fibroblast growth factor, bFGF (Abraham *et al*. (1986), *EMBO J.,* 5:2523-2528); acidic fibroblast growth factor, aFGF (Jaye *et al*. (1986), *Science,* 233:541-545); int-2 gene product, int-2 (Dickson & Peters (1987), *Nature*, 326:833); hst/kFGF (Delli-Bovi *et al.* (1987), *Cell,* 50:729-737 and Yoshida *et al.* (1987), *Proc. Natl. Acad. Sci. USA,* 84:7305-7309); FGF-5 (Zhan *et al.* (1988), *Mol. Cell. Biol.,* 8:3487-3495); FGF-6 (Marics *et al*. (1989), *Oncogene,* 4:335-340); keratinocyte growth factor (Finch *et al*. (1989), *Science,* 24:752-755) and hisactophilin (Habazzettl *et al*. (1992), *Nature,* 359:855-858).

Among the FGF family of proteins, keratinocyte growth factor ("KGF") is a unique effector of non-fibroblast epithelial (particularly keratinocyte) cell proliferation derived from mesenchymal tissues. The term "native KGF" refers to a natural human (hKGF) or recombinant (rKGF) polypeptide (with or without a signal sequence) as depicted by the amino acid sequence presented in SEQ ID NO:2 or an allelic variant thereof. [Unless otherwise indicated, amino acid numbering for molecules described herein shall correspond to that presented for the mature form of the native molecule (i.e., minus the signal sequence), as depicted by amino acids 32 to 194 of SEQ ID NO:2.]

Native KGF may be isolated from natural human sources (hKGF) or produced by recombinant DNA techniques (rKGF) (Finch *et al*. (1989), *supra;* Rubin *et al*. (1989), *supra;* Ron *et al*. (1993), *The Journal of Biological Chemistry,* 268(4):2984-2988; and Yan *et al*. (1991), *In Vitro Cell. Dev. Biol.,* 27A:437-438).

It is known that native KGF is relatively unstable in the aqueous state and that it undergoes chemical and physical degradation resulting in a loss of biological activity during processing and storage (Chen *et al*. (1994), *Pharmaceutical Research,* 11:1582-1589). Native KGF is prone also to aggregation at elevated temperatures and it becomes inactivated under acidic conditions (Rubin *et al.* (1989), *Proc. Natl. Acad. Sci. USA,* 86:802-806). Aggregation of native KGF in aqueous solution also results in inactivated protein. This is disadvantageous because such loss of activity makes it impractical to store aqueous formulations of native KGF proteins for extended periods of time or to administer the protein over extended periods. Moreover, this is particularly problematic when preparing pharmaceutical formulations, because aggregated proteins have been known to be immunogenic (Cleland *et al.* (1993), *Crit. Rev. Therapeutic Drug Carrier Systems,* 10:307-377; Robbins *et al.* (1987), *Diabetes*, 36:838-845; and Pinckard *et al.* (1967), *Clin. Exp. Immunol*., 2:331-340).

Recombinant DNA technology has been utilized to modify the sequences of various FGF family members. For example, bFGF and aFGF have been modified by deleting or substituting positively-charged residues, which are important for heparin binding with neutral or negatively-charged amino acids. It was reported that the modified molecules resulted in reduced heparin binding activity. Accordingly, it was taught that the amount of modified molecule sequestered by heparin and/or heparin-like molecules in a patient would be reduced, thereby increasing potency as more of the FGF will reach its targeted receptor (EP 0 298 723).

In order to improve or otherwise alter one or more of the characteristics of native KGF, protein engineering may be employed. Ron *et al.* (1993), *J*. *Biol. Chem.,* 268(4):2984-2988 reported modified KGF polypeptides having 3, 8, 27, 38 or 49 amino acids deleted from the N-terminus. Those polypeptides missing 3, 8, or 27 N-terminal residues retained heparin binding ability; the others did not. Also, the polypeptides missing 3 and 8 residues were reported as being fully active, whereas the form missing 27 residues was 10-20 fold less mitogenic, and the forms lacking 38 or 49 amino acids did not have mitogenic activity. The stability of the modified KGF polypeptides was not discussed or otherwise reported.

Published PCT application no. 95/08630 reported on surface loop structural analogs of fibroblast growth factors, which analogues retained heparin binding capability. There is no disclosure pertaining to the stability of these analogues.

Published PCT application no. 90/08771, *supra,* also reported the production of a chimeric protein wherein about the first 40 N-terminal amino acids of mature form of native KGF were combined with the C-terminal portion (about 140 amino acids) of aFGF. The chimera was reported to target keratinocytes like KGF, but it lacked susceptibility to heparin, a characteristic of aFGF but not KGF. The stability of the chimera was not discussed or otherwise reported.

Thus, the literature has not reported a modified KGF molecule having significantly improved stability relative to native KGF. Moreover, the literature has not reported sufficient teachings or evidence to provide a reasonable expectation of successfully generating KGF molecules with such desirable characteristics.

It is not currently possible to predict the characteristics of a protein based upon the knowledge of only its primary structure. For example, the mitogenic activity of aFGF is substantially increased in the presence of heparin, but the mitogenic activity of bFGF in the presence of heparin is only minimally increased, despite the fact that heparin tightly binds to bFGF [(Burgess and Maciag (1989), *Annu. Rev. Biochem.,* 58:575-606; Schreiber, *et al.* (1985), *Proc Natl. Acad. Sci. USA,* 82:6138-6142; and Gospodarowizc and Cheng (1986), *J. Cell Physiol.,* 128:475-485); and PCT 90/00418)]. In contrast, thymidine incorporation by BALB/MK cells is inhibited when heparin is included with KGF in the culture medium.

Generally, the effects upon biological activity of any amino acid change upon the protein will vary depending upon a number of factors, including the three-dimensional structure of the protein and whether or not the modification is to either the heparin binding region or the receptor binding region on the primary sequence of the protein. As neither the three-dimensional structure nor the heparin binding region and the receptor binding region on the primary sequence of native KGF has been published, the knowledge within the art does not permit generalization about the effects of amino acid modifications to native KGF based upon the effects of amino acid modifications on even commonly categorized proteins.

It is the object of this invention to provide polypeptide analogs of KGF and nucleic acid molecules encoding such analogs that exhibit enhanced stability (e.g., when subjected to typical pH, thermal and/or other storage conditions) as compared to native KGF.

### Summary of the Invention

The present invention provides novel, biologically active polypeptide analogs of native keratinocyte growth factor termed "KGF," wherein native KGF corresponds to the following sequence 
Cys
1
Asn Asp Met Thr Pro Glu Gln Met Ala Thr Asn Val Asn Cys Ser Ser
            5                   10                  15
Pro Glu Arg His Thr Arg Ser Tyr Asp Tyr Met Glu Gly Gly Asp Ile
        20                  25                  30
Arg Val Arg Arg Leu Phe Cys Arg Thr Gln Trp Tyr Leu Arg Ile Asp
    35                  40                  45
Lys Arg Gly Lys Val Lys Gly Thr Gln Glu Met Lys Asn Asn Tyr Asn
50 50 60 65
Ile Met Glu Ile Arg Thr Val Ala Val Gly Ile Val Ala Ile Lys Gly
                70                  75                  80
Val Glu Ser Glu Phe Tyr Leu Ala Met Asn Lys Glu Gly Lys Leu Tyr
            85                  90                  95
Ala Lys Lys Glu Cys Asn Glu Asp Cys Asn Phe Lys Glu Leu Ile Leu
        100                 105                 110
Glu Asn His Tyr Asn Thr Tyr Ala Ser Ala Lys Trp Thr His Asn Gly
    115                 120                 125
Gly Glu Met Phe Val Ala Leu Asn Gln Lys Gly Ile Pro Val Arg Gly
130                 135                 140                 145
Lys Lys Thr Lys Lys Glu Gln Lys Thr Ala His Phe Leu Pro Met Ala
                150                 155                 160
Ile Thr
 said analogs having an improved stability wherein the analogs comprise the amino acid sequence of KGF having a charge-change by the deletion and/or substitution of one or more of amino acid residues 41-154 of the above sequence to effect a reduced positive charge as compared with native KGF, optionally further having a deletion of the first 15 to 24 amino acids of the N-terminal native KGF, having residues corresponding to Cys (1) and Cys (15) replaced or deleted, having an N-terminal methionine or having a signal sequence, with the proviso that the amino acid sequence 123-131 of the above sequence is not substituted by any one of the amino acid sequences selected from the group of DLYQG and AKYEG.

Surprisingly, it has been discovered that by deleting or replacing the more positively charged residues with neutral or negatively charged residues (i.e. substituting positively charged residues by neutral or negatively charged residues, or neutral residues by negatively charged residues) of a KGF molecule (i.e., parent molecule), the resultant KGF analog has improved stability as compared to the parent molecule. Preferably, in addition to having increased stability, the invention is directed to those analogs which also exhibit full biological activity (i.e., at least substantially similar receptor binding or affinity) as compared to native KGF.

In another aspect of the invention, purified and isolated nucleic acid molecules encoding the various biologically active polypeptide analogs of KGF are described. In one embodiment, such nucleic acids comprise DNA molecules cloned into biologically functional plasmid or viral vectors. In another embodiment, nucleic acid constructs may then be utilized to stably transform a procaryotic or eucaryotic host cell. In still another embodiment, the invention involves a process wherein either a procaryotic (preferably *E. coli)* or eucaryotic host cell stably transformed with a nucleic acid molecule is grown under suitable nutrient conditions in a manner allowing the expression of the KGF analog. Following expression, the resultant recombinant polypeptide can be isolated and purified.

A further aspect of the invention concerns pharmaceutical formulations comprising a therapeutically effective amount of a KGF analog and an acceptable pharmaceutical carrier. Such formulations will be useful in treating patients afflicted with epithelial diseases and injuries.

In this vein, another aspect relates to the use of a KGF analog for preparing a medicament for the treatment of disease requiring the stimulation of the production of non-fibroblast epithelial cells. Such epithelial cells include various adnexal cells, pancreatic cells, liver cells, and mucosal epithelium in the respiratory and gastrointestinal tracts.

### Brief Description of the Figures

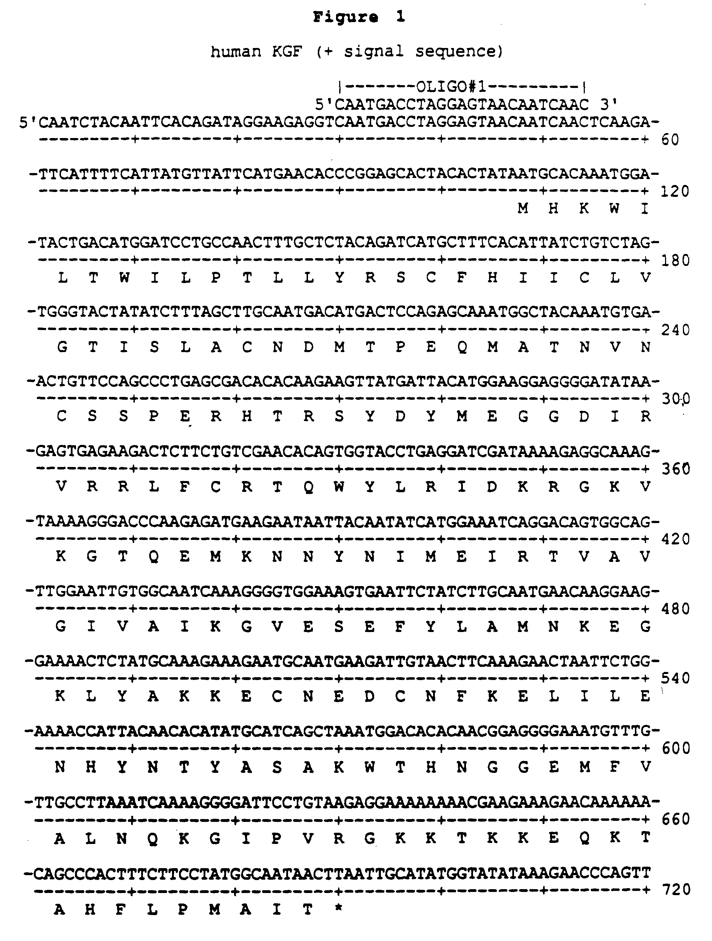

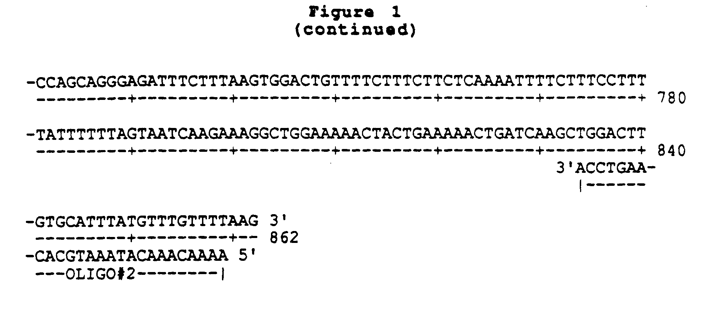

Figure 1 shows the nucleotide (SEQ ID NO:1) and amino acid (SEQ ID NO:2) sequences of native KGF (the nucleotides encoding the mature form of native KGF are depicted by bases 201 to 684 of SEQ ID NO:1 and the mature form of KGF is depicted by amino acid residues 32 to 194 of SEQ ID NO:2).

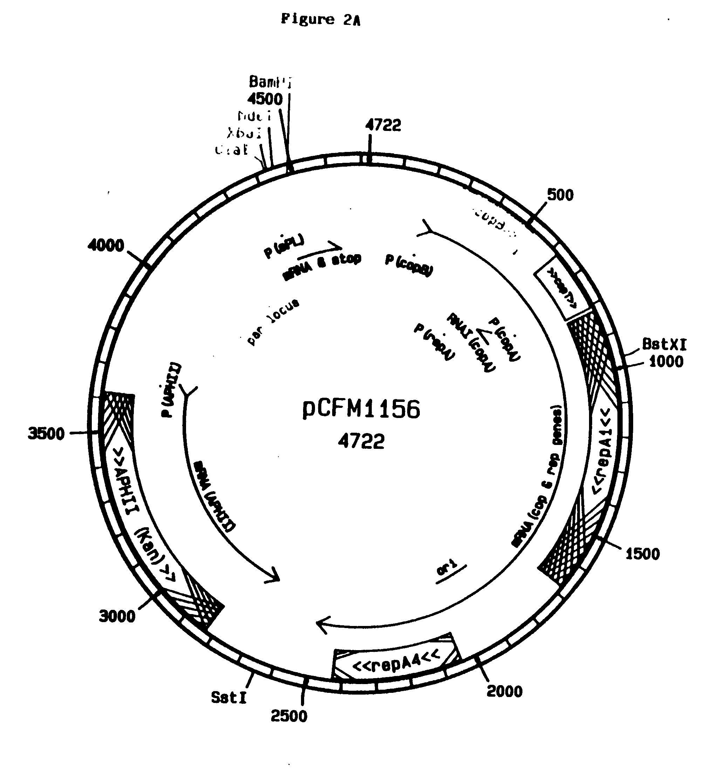

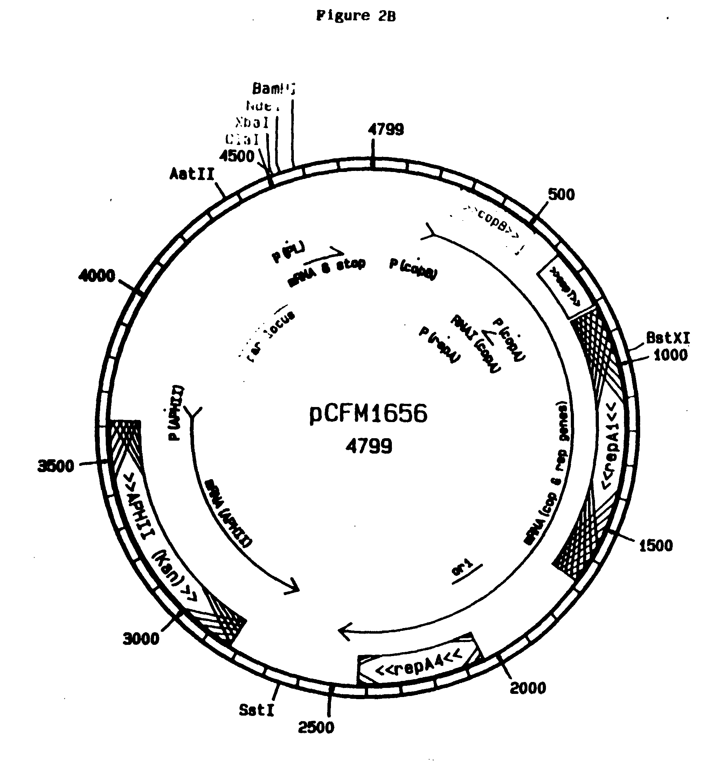

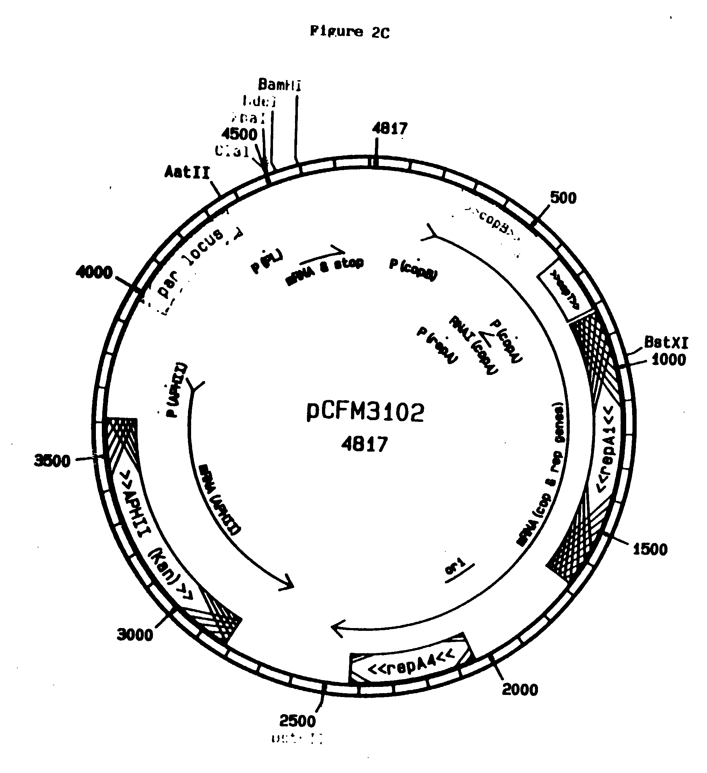

Figures 2A, 2B and 2C show the plasmid maps of pCFM1156, pCFM1656 and pCFM3102, respectively.

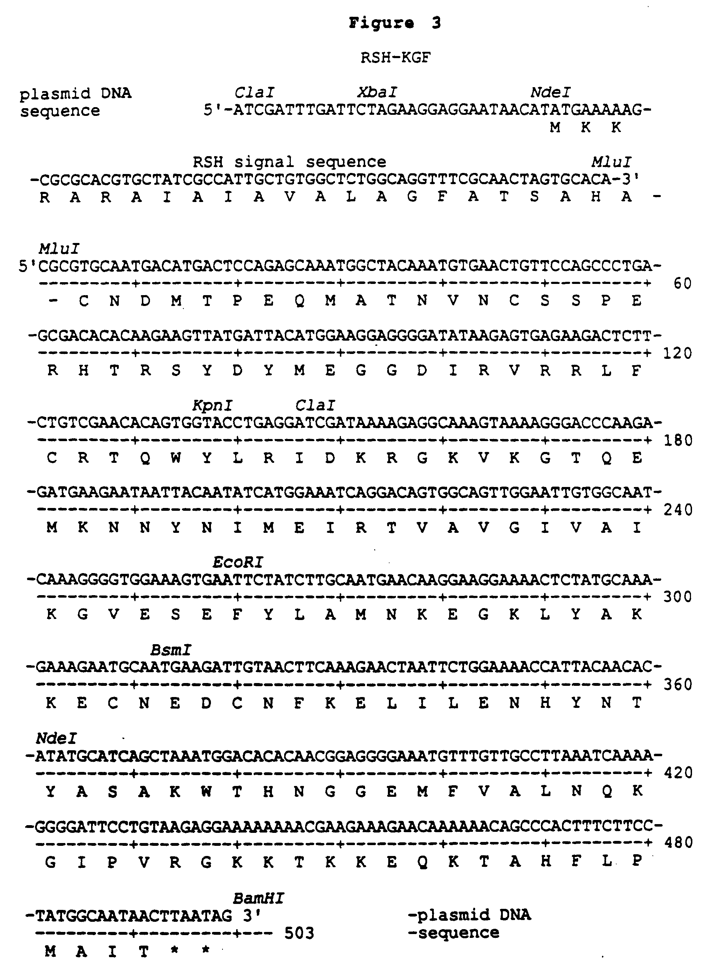

Figure 3 shows the nucleotide (SEQ ID NO:3) and amino acid (SEQ ID NO:4) sequences of the construct RSH-KGF.

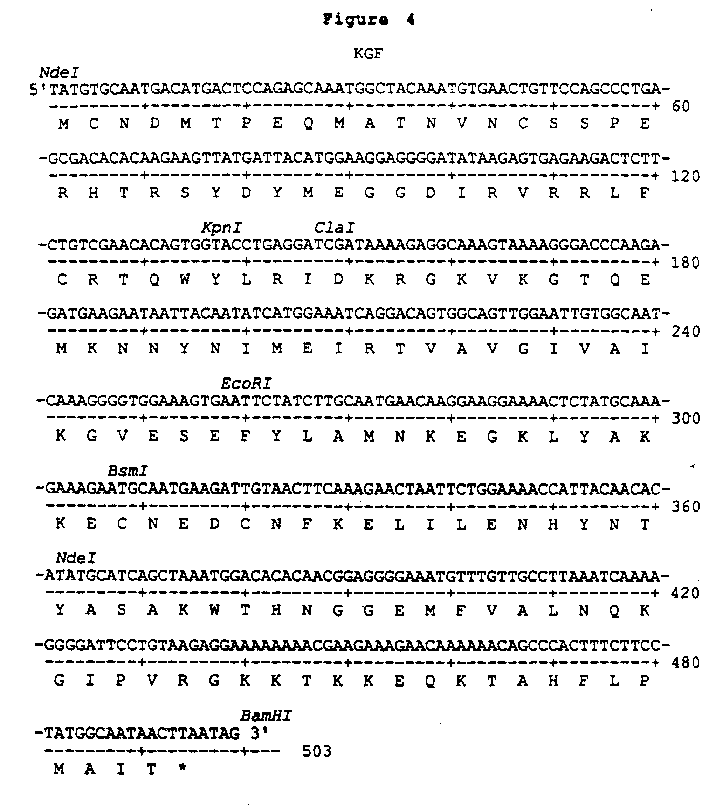

Figure 4 shows the nucleotide (SEQ ID NO:5) and amino acid (SEQ ID NO:6) sequences of the construct contained in plasmid KGF.

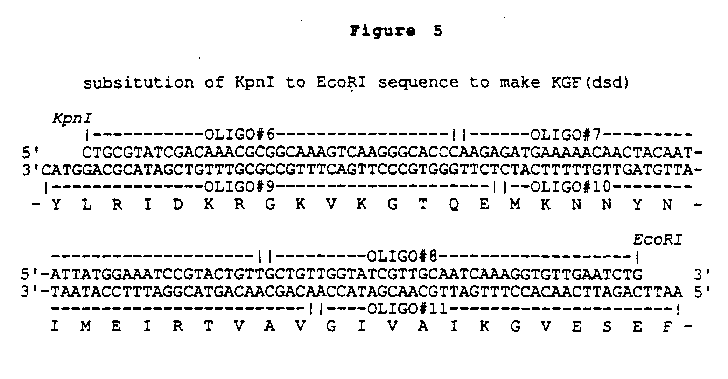

Figure 5 shows the chemically synthesized OLIGOs (OLIGO#6 through OLIGO#11; SEQ ID NO:12-17, respectively) used to substitute the DNA sequence between a *KpnI* site and an *EcoRI* site (from amino acid positions 46 to 85 of SEQ ID No:6) in the construct contained in plasmid KGF to produce the construct in plasmid KGF(dsd).

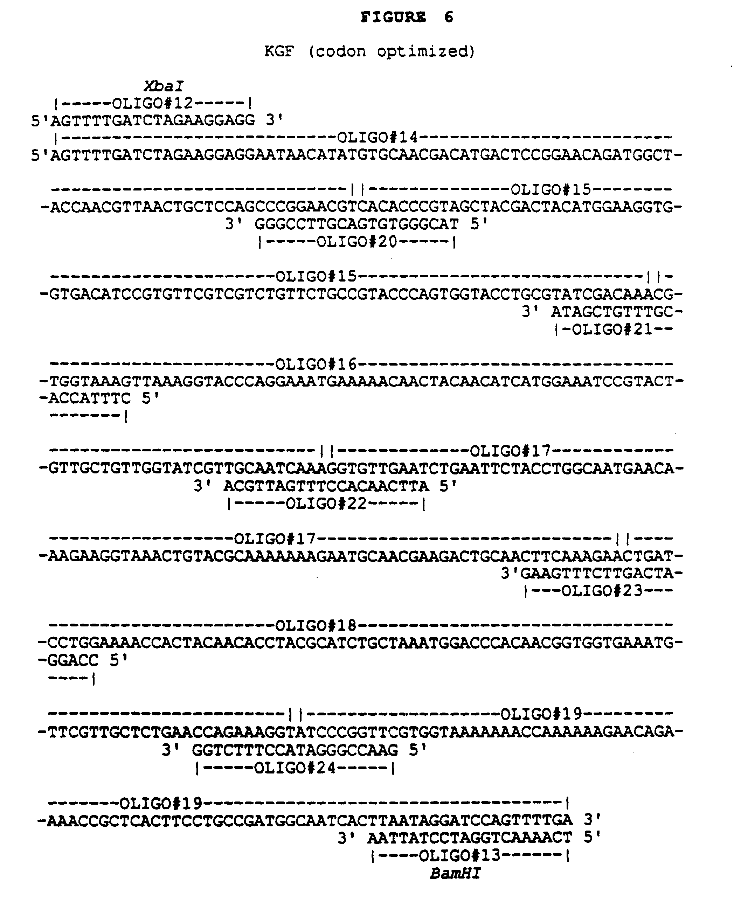

Figure 6 shows the chemically synthesized OLIGOs (OLIGO#12 through OLIGO#24; SEQ ID NO:18-30, respectively) used to construct KGF (codon optimized).

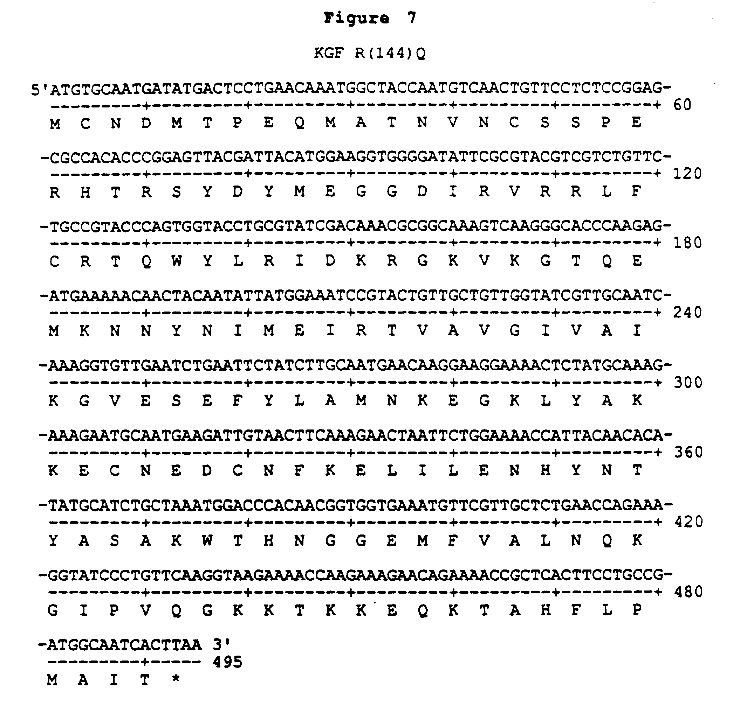

Figure 7 shows the nucleotide (SEQ ID NO:31) and amino acid sequences (SEQ ID NO:32) of R(144)Q, a KGF analog having a substitution of glutamine for arginine at amino acid position 144 of native KGF, wherein the initial methionine in the sequence should be considered residue number "0".

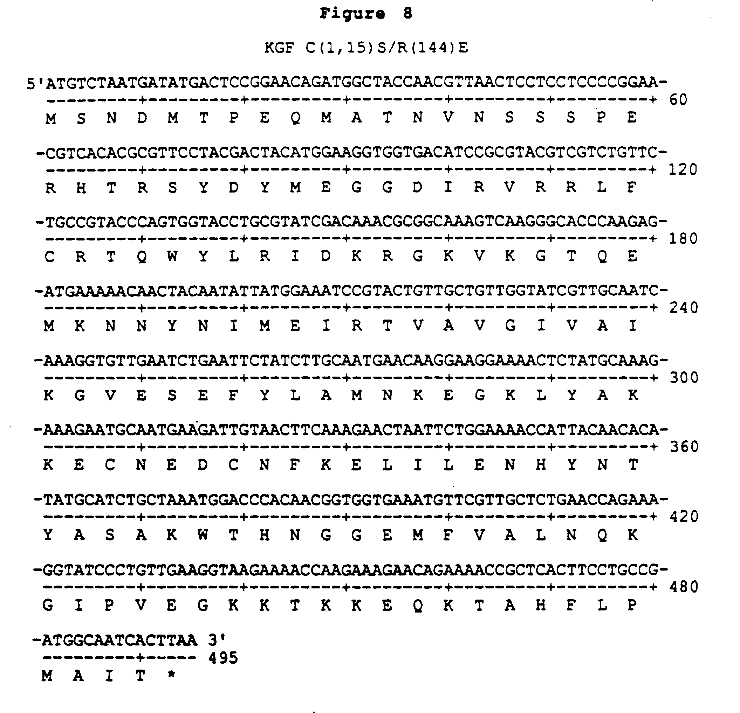

Figure 8 shows the nucleotide (SEQ ID NO:33) and amino acid sequences (SEQ ID NO:34) of C(1,15)S/R(144)E, a KGF analog having substitutions of serine for cysteine at amino acid positions 1 and 15 and a substitution of glutamic acid for arginine at amino acid position 144 of native KGF, wherein the initial methionine in the sequence should be considered residue number "0".

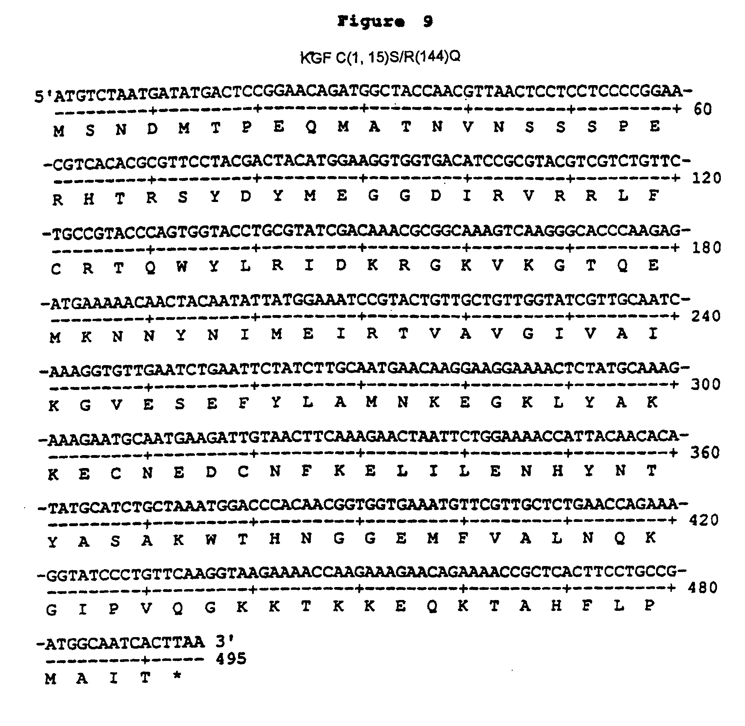

Figure 9 shows the nucleotide (SEQ ID NO:35) and amino acid (SEQ ID NO:36) sequences of C(1,15)S/R(144)Q, a KGF analog having substitutions of serine for cysteine at amino acid positions 1 and 15 and a substitution of glutamine for arginine at amino acid position 144 of native KGF, wherein the initial methionine in the sequence should be considered residue number "0".

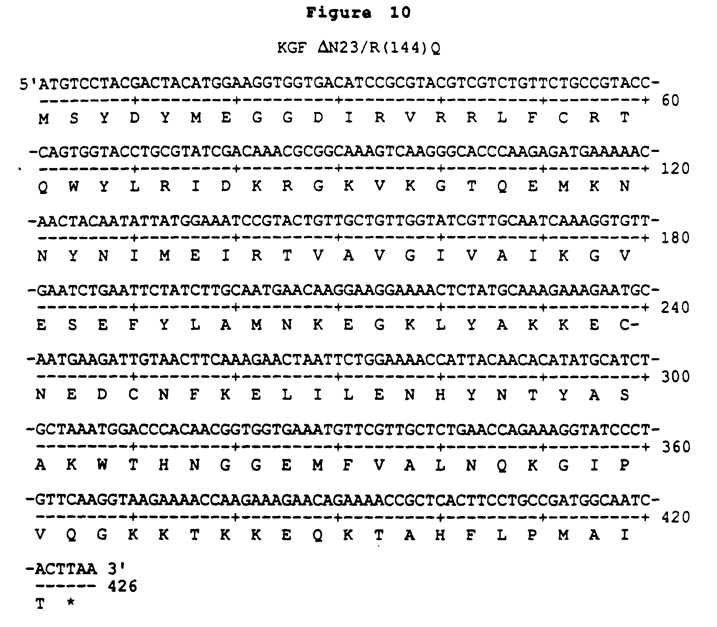

Figure 10 shows the nucleotide (SEQ ID NO:37) and amino acid (SEQ ID NO:38) sequences of ΔN23/R(144)Q, a KGF analog having a deletion of the first 23 amino acids of the N-terminus and a substitution of glutamine for arginine at amino acid position 144 of native KGF, wherein the initial methionine in the sequence should be considered residue number "0".

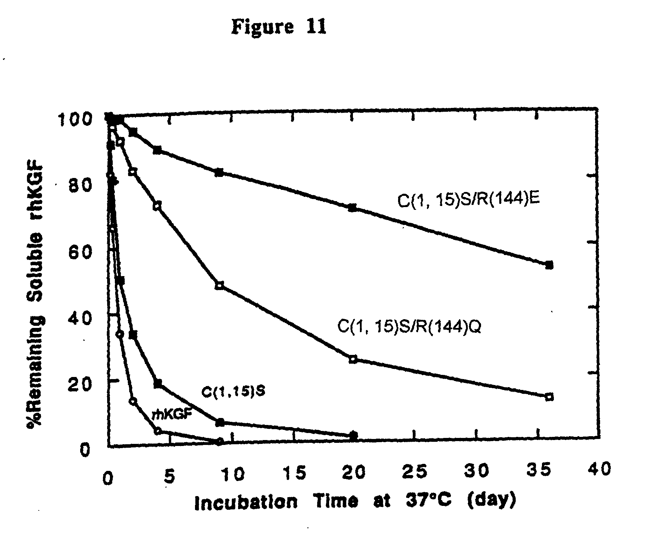

Figure 11 shows the amount of soluble protein, determined by size exclusion HPLC, as a function of incubation time at 37°C.

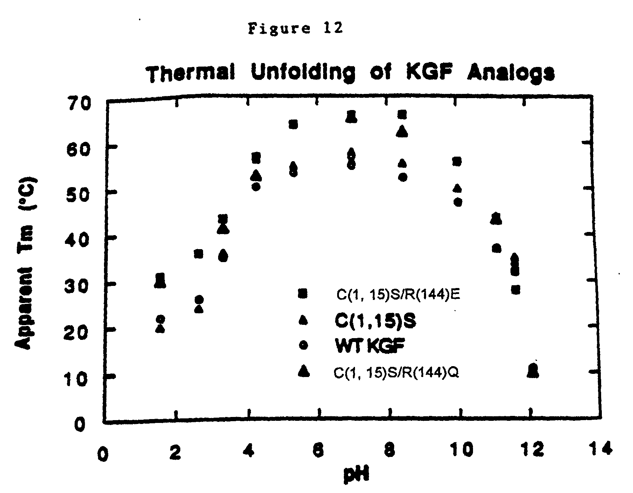

Figure 12 shows the estimated melting temperature (Tₘ) as a function of pH for native KGF, C(1,15)S,C(1,15)S/R(144)Q and C(1,15)S/R(144)E.

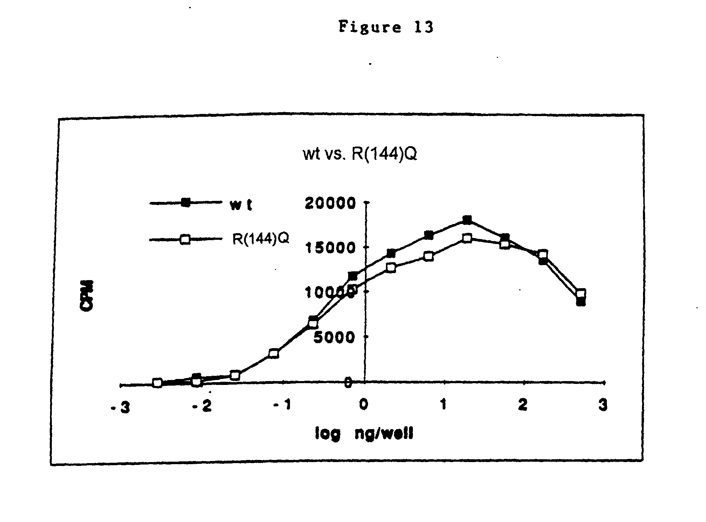

Figure 13 shows a typical profile of mitogenic activity of R(144)Q, determined by measuring the incorporation of [³H]-Thymidine during DNA synthesis and by comparing it to a native KGF standard curve.

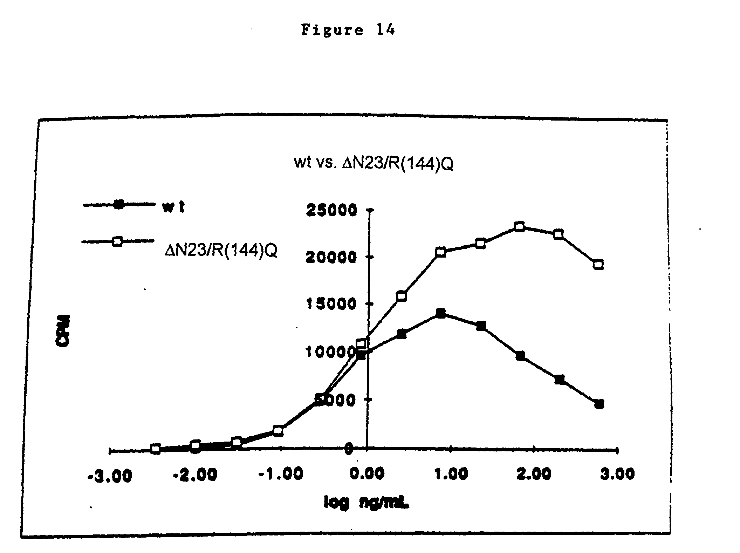

Figure 14 shows a typical profile of the mitogenic activity of ΔN23/R(144)Q, determined by measuring the incorporation of [³H]-Thymidine during DNA synthesis and by comparing it to a native KGF standard curve.

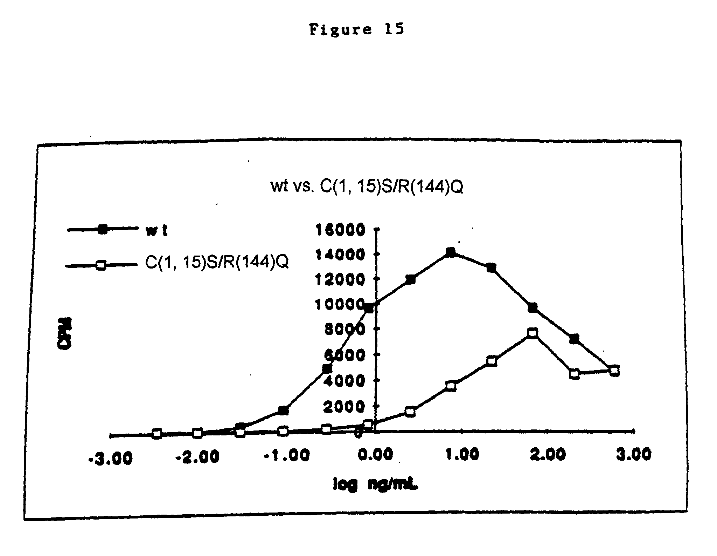

Figure 15 shows a typical profile of the mitogenic activity of C(1,15)S/R(144)Q, determined by measuring the incorporation of [³H]-Thymidine during DNA synthesis and by comparing it to a native KGF standard curve.

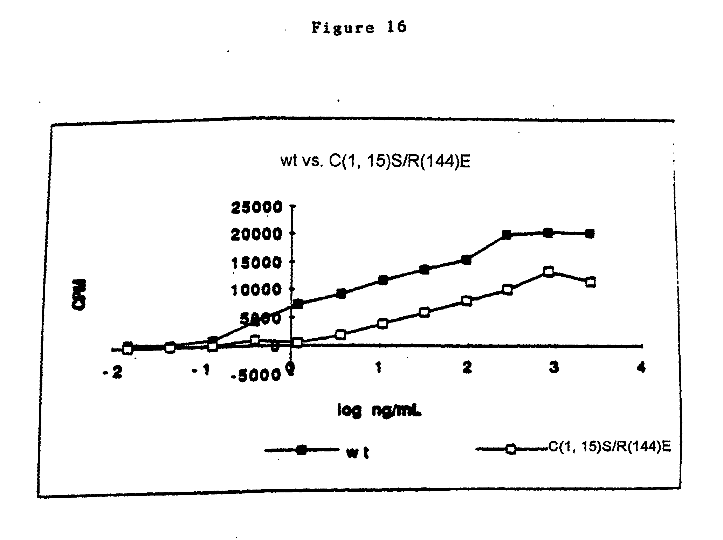

Figure 16 shows a typical profile of the mitogenic activity of C(1,15)S/R(144)E, determined by measuring the incorporation of [³H]-Thymidine during DNA synthesis and by comparing it to a native KGF standard curve.

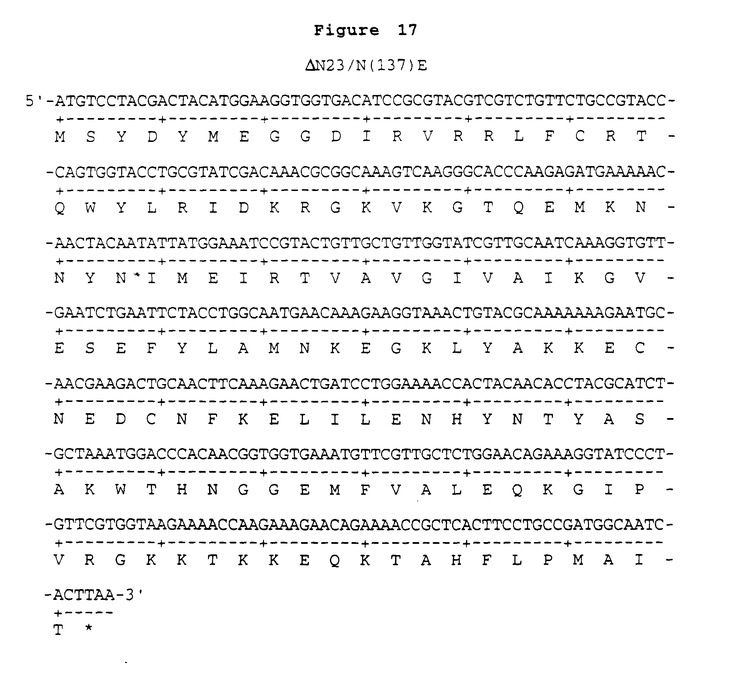

Figure 17 shows the nucleotide (SEQ ID NO:41) and amino acid (SEQ ID NO:42) sequences of ΔN23/N(137)E, a KGF analog having a deletion of the first 23 amino acids of the N-terminus and a substitution of glutamic acid for asparagine at amino acid position 137 of native KGF, wherein the initial methionine in the sequence should be considered residue number "0".

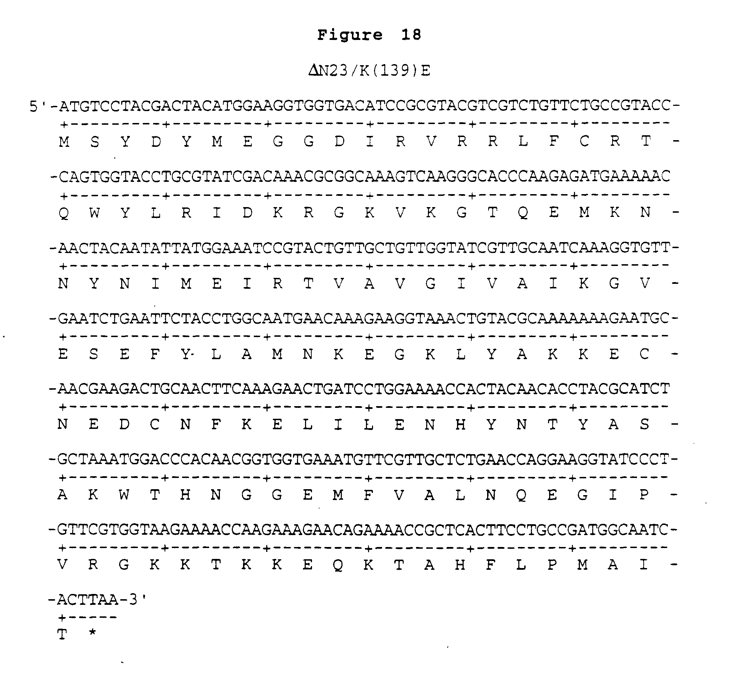

Figure 18 shows the nucleotide (SEQ ID NO:43) and amino acid (SEQ ID NO:44) sequences of ΔN23/K(139)E, a KGF analog having a deletion of the first 23 amino acids of the N-terminus and a substitution of glutamic acid for lysine at amino acid position 139 of native KGF, wherein the initial methionine in the sequence should be considered residue number "0".

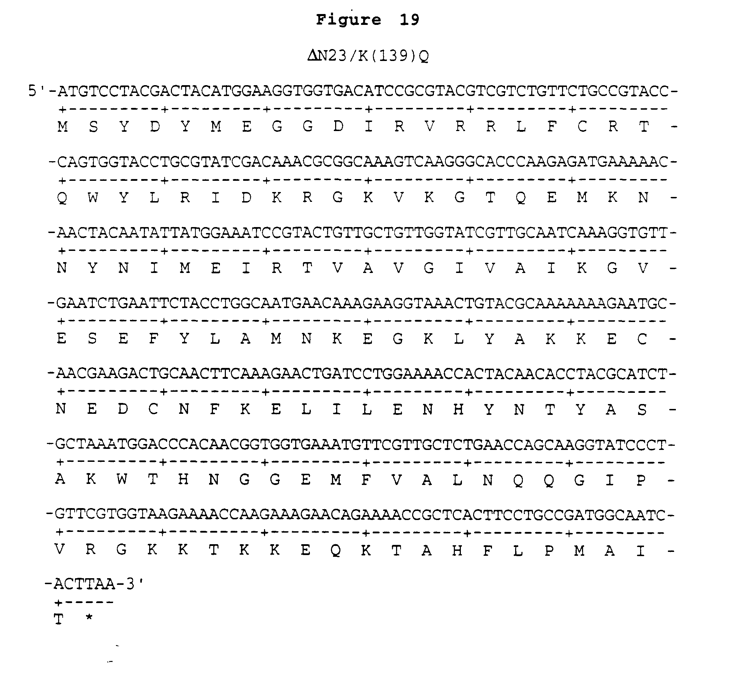

Figure 19 shows the nucleotide (SEQ ID NO:45) and amino acid (SEQ ID NO:46) sequences of ΔN23/K(139)Q, a KGF analog having a deletion of the first 23 amino acids of the N-terminus and a substitution of glutamine for lysine at amino acid position 139 of native KGF, wherein the initial methionine in the sequence should be considered residue number "0".

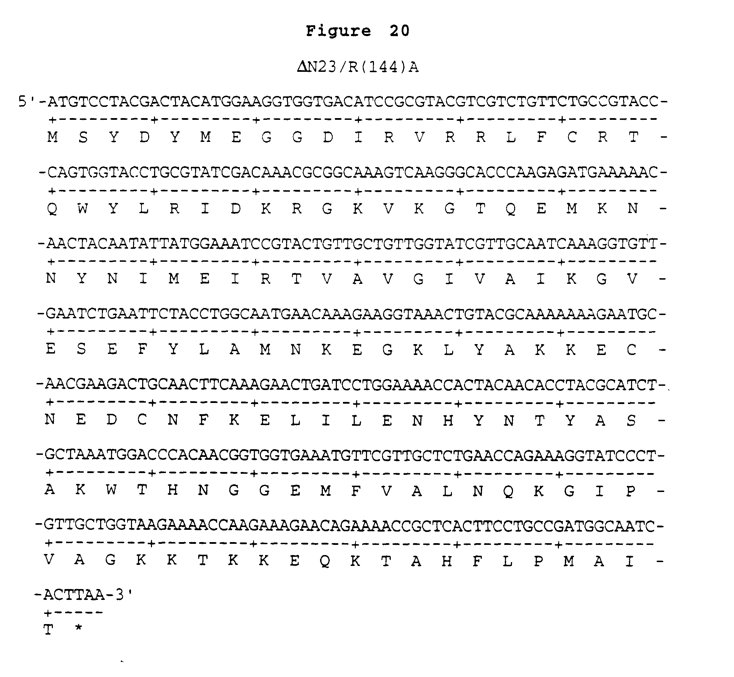

Figure 20 shows the nucleotide (SEQ ID NO:47) and amino acid (SEQ ID NO:48) sequences of ΔN23/R(144)A, a KGF analog having a deletion of the first 23 amino acids of the N-terminus and a substitution of alanine for arginine at amino acid position 144 of native KGF, wherein the initial methionine in the sequence should be considered residue number "0".

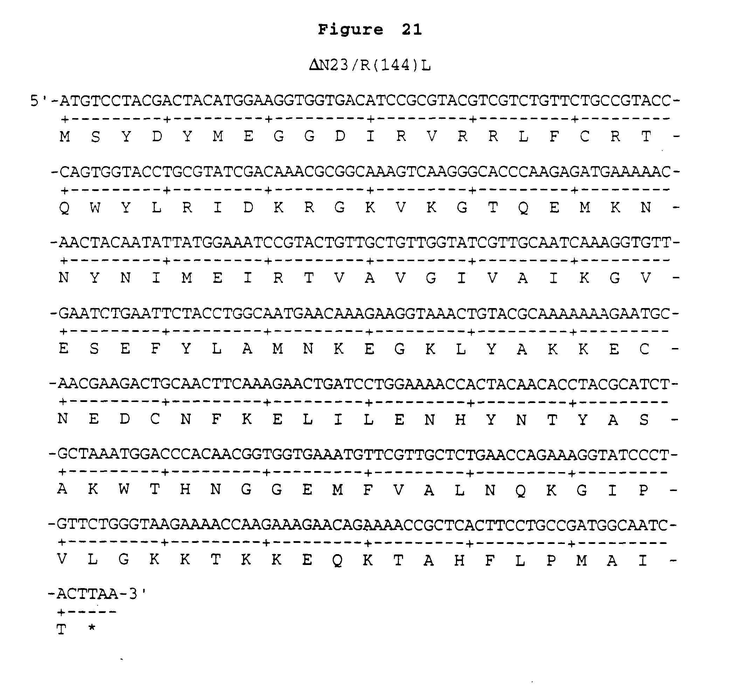

Figure 21 shows the nucleotide (SEQ ID NO:49) and amino acid (SEQ ID NO:50) sequences of ΔN23/R(144)L, a KGF analog having a deletion of the first 23 amino acids of the N-terminus and a substitution of leucine for arginine at amino acid position 144 of native KGF, wherein the initial methionine in the sequence should be considered residue number "0".

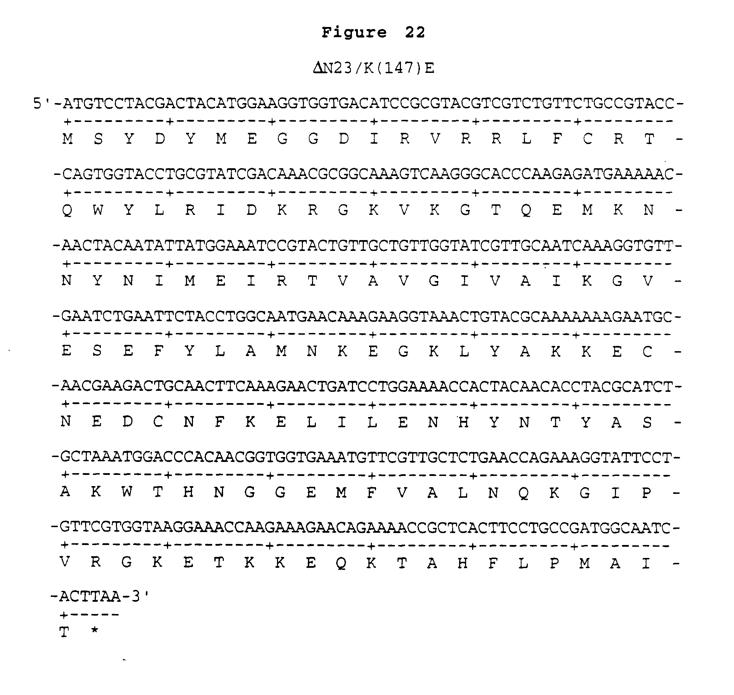

Figure 22 shows the nucleotide (SEQ ID NO:51) and amino acid (SEQ ID NO:52) sequences of ΔN23/K(147)E, a KGF analog having a deletion of the first 23 amino acids of the N-terminus and a substitution of glutamic acid for lysine at amino acid position 147 of native KGF, wherein the initial methionine in the sequence should be considered residue number "0".

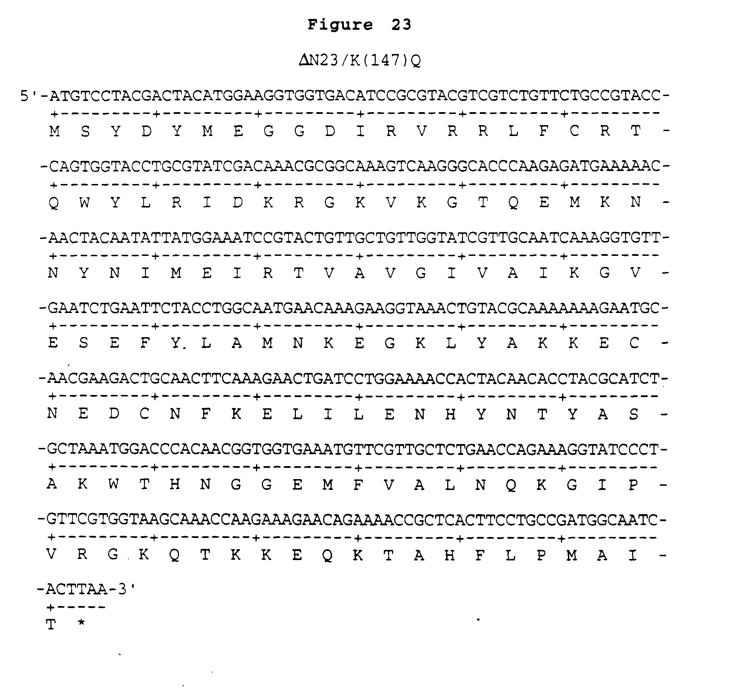

Figure 23 shows the nucleotide (SEQ ID NO:53) and amino acid (SEQ ID NO:54) sequences of ΔN23/K(147)Q, a KGF analog having a deletion of the first 23 amino acids of the N-terminus and a substitution of glutamine for lysine at amino acid position 147 of native KGF, wherein the initial methionine in the sequence should be considered residue number "0".

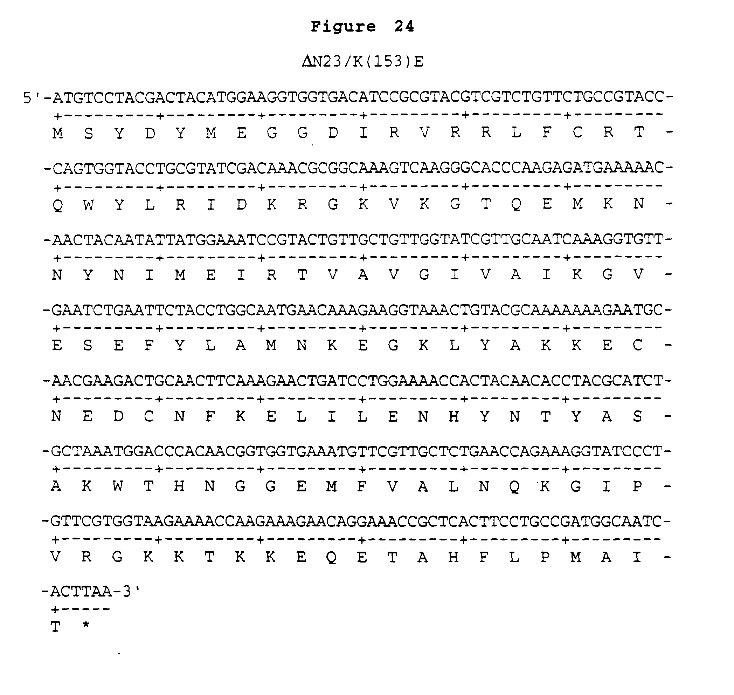

Figure 24 shows the nucleotide (SEQ ID NO:55) and amino acid (SEQ ID NO:56) sequences of ΔN23/K(153)E, a KGF analog having a deletion of the first 23 amino acids of the N-terminus and a substitution of glutamic acid for lysine at amino acid position 153 of native KGF, wherein the initial methionine in the sequence should be considered residue number "0".

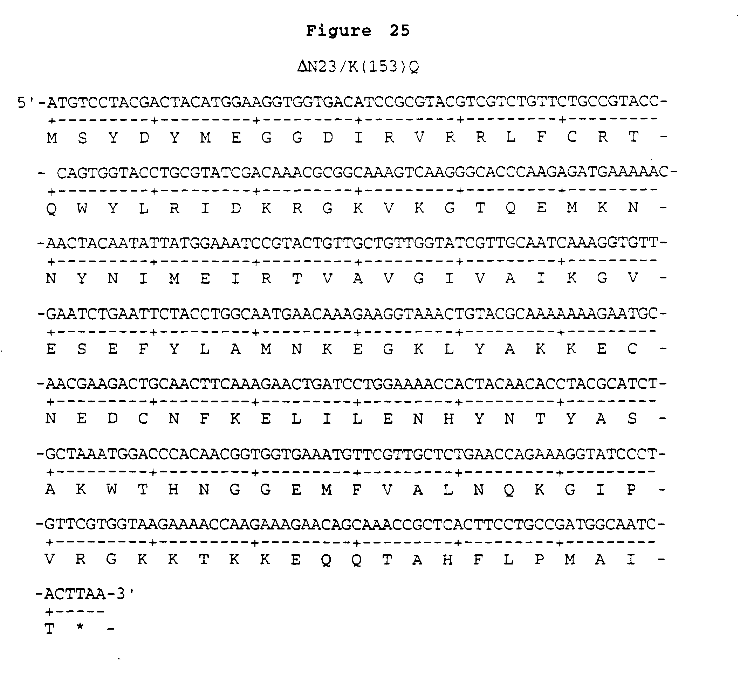

Figure 25 shows the nucleotide (SEQ ID NO:57) and amino acid (SEQ ID NO:58) sequences of ΔN23/K(153)Q, a KGF analog having a deletion of the first 23 amino acids of the N-terminus and a substitution of glutamine for lysine at amino acid position 153 of native KGF, wherein the initial methionine in the sequence should be considered residue number "0".

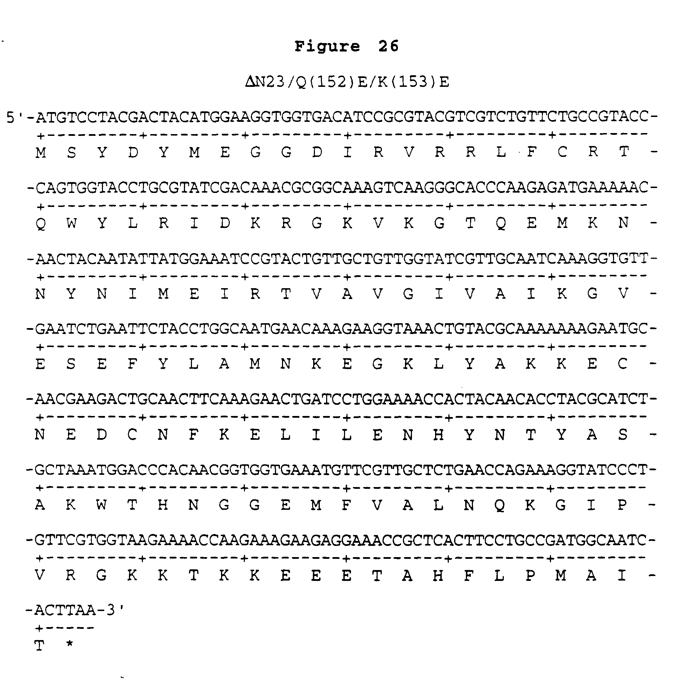

Figure 26 shows the nucleotide (SEQ ID NO:59) and amino acid (SEQ ID NO:60) sequences of ΔN23/Q(152)E/K(153)E, a KGF analog having a deletion of the first 23 amino acids of the N-terminus and a substitution of glutamic acid for glutamine at amino acid position 152 of native KGF and glutamic acid for lysine at amino acid position 153 of native KGF, wherein the initial methionine in the sequence should be considered residue number "0".

### Detailed Description

In accordance with the present invention, novel analogs of KGF are provided. The KGF analogs are preferably produced by deleting or substituting one or more specific, positively-charged residues in KGF.

The KGF analogs have, among other properties, an improved stability under at least one of a variety of purification and/or storage conditions. For example, the KGF analogs will generally be purified in a greater yield of soluble, correctly folded protein. Moreover, once the material is purified, it will be more stable to pH, temperature, etc. as compared to the stability of the parent molecule. As described in the Examples section below (modified by the substitution of Gln and Glu for arginine at position 144 [R(144)Q and R(144)E, respectively] and in some instances modified at the N-terminus as well) exhibit, relative to native KGF, (1) a 35 to 37.2 day increase of half-life upon storage at 37°C, (2) a 7.5-9.5% higher thermal melting temperatures over the course of thermal unfolding, and (3) an increase in Tₘ over a range of pH values.

Although not intended to be bound by theory, a possible reason for the enhanced stability of the R(144)Q and R(144)E may be due to a reduction in overall charge density of a cluster of basic residues, which is inherently unstable due to charge repulsion, in the absence of heparin. The results set forth below suggest that the arginine residue at position 144 may correspond to a residue in bFGF, as determined by X-ray crystallography, which is reported to be within or near a cluster of basic residues that mediate heparin binding (Ago, *et al.* (1991), *J. Biochem.,* 110:360-363; and Eriksson *et al.* (1993), *Protein Science,* 2:1274-1284).

Native KGF contains 46 charged residues, 27 of which carry a positive charge. In view of the results obtained with the KGF analogs, a comparison of the native KGF primary sequence with the primary sequence of bFGF suggests that some of the 27 positively charged residues form a cluster similar to a cluster found in the tertiary structure of bFGF. Depending on the location of such residues in the protein's three-dimensional structure, substitution of one or more of these clustered residues with amino acids carrying a negative or neutral charge may alter the electrostatic interactions of adjacent residues and may be useful to achieve increased stability.

Thus other analogs, in addition to the preferred R(144)Q specifically set forth herein, are contemplated by the present invention. As used in this invention, a "KGF analog" or a "polypeptide analog of KGF" shall mean charge-change polypeptides wherein one or more of amino acid residues 41-154 (amino acids 72-185 of SEQ ID NO:2), specifically including amino acid residues 123-133 (amino acids 154-164 of SEQ ID NO:2), are deleted or substituted with a neutral residue or negatively charged residue selected to effect a protein with a reduced positive charge. Preferred residues for modification are Arg⁴¹, Gln⁴³, Lys⁵⁵, Lys⁹⁵, Lys¹²⁸, Asn¹³⁷, Gln¹³⁸, Lys¹³⁹, Arg¹⁴⁴, Lys¹⁴⁷, Gln¹⁵², Lys¹⁵³ or Thr¹⁵⁴, with Gln¹³⁸, Lys¹³⁹, Arg¹⁴⁴, Lys¹⁴⁷, Gln¹⁵² or Lys¹⁵³ being more preferred and Arg¹⁴⁴ being most preferred. Preferred amino acids for substitution include glutamic acid, aspartic acid, glutamine, asparagine, glycine, alanine, valine, leucine, isoleucine, serine and threonine, with glutamic acid, glutamine, aspartic acid, asparagine and with alanine being particularly preferred.

Preferred polypeptide analogs according to the present invention are those wherein the deleted or substituted amino acid residues are selected from the arginine residue at amino acid position 41, the glutamine residue at amino acid position 43, the lysine residue at amino acid position 55, the lysine residue at amino acid position 95, the asparagine residue at amino acid position 137, the glutamine residue at amino acid position 138, the lysine residue at amino acid position 139, the arginine residue at amino acid position 144, the lysine residue at amino acid position 147, the glutamine residue at amino acid position 152, the lysine residue at amino acid position 153, or the threonine residue at amino acid position 154 of the sequence depicted in claim 1.

Even more preferred polypeptide analogs of the present invention are those selected from R(144)Q, C(1,15)S/R(144)E, ΔN23/R(144)Q, ΔN23/N(137)E, ΔN23/K(139)E, ΔN23/K(139)Q, ΔN23/R(144)A, ΔN23/R(144)E (a KGF analog having a deletion of the first 23 amino acids of the N-terminus and a substitution of glutamic acid for arginine at amino acid position 144 of native KGF), ΔN23/R(144)L, ΔN23/K(127)E, ΔN23/K(137)Q, ΔN23/K(153)E, ΔN23/K(153)Q, or ΔN23/Q(152)E/K(153)E.

Any modification should give consideration to minimizing charge repulsion in the tertiary structure of the molecule; most preferably the analog will have increased stability compared with the parent molecule. Obviously, the deletions or substitutions should not be so numerous nor be made to residues of such close proximity so as to set up charge repulsion between two negatively-charged residues.

When the KGF analogs are biologically generated, *i.e*., are the products of cellular expression as opposed to the products of solid state synthesis, proteolytic or enzymatic derivatization of naturally-occurring products, etc., the nucleic acids encoding such polypeptides will differ in one or more nucleotides as compared to the native KGF nucleotide sequence. Such polynucleotides may be expressed and the resultant polypeptide purified by any one of a number of recombinant technology methods known to those skilled in the art.

DNA sequences coding for all or part of the KGF analogs may include, among other things, the incorporation of codons "preferred" for expression in selected host cells (e.g., *"E. coli* expression codons"); the provision of sites for cleavage by restriction enzymes; and the provision of additional initial, terminal, or intermediate nucleotide sequences (e.g., as an initial methionine amino acid residue for expression in *E. coli* cells), to facilitate construction of readily expressed vectors.

The present invention also provides for recombinant molecules or vectors for use in the method of expression of the polypeptides. Such vectors may be comprised of DNA or RNA and can be circular, linear, single-stranded or double-stranded in nature and can be naturally-occurring or assemblages of a variety of components, be they naturally-occurring or synthetic.

Many examples of such expression vectors are known. The components of the vectors, e.g. replicons, selection genes, enhancers, promoters, and the like, may be obtained from natural sources or synthesized by known procedures. In each case, expression vectors useful in this invention will contain at least one expression control element functionally associated with the inserted nucleic acid molecule encoding the KGF polypeptide analog. This control element is responsible for regulating polypeptide expression from the nucleic acid molecules of the invention. Useful control elements include, for example, the *lac* system, the *trp* system, the operators and promoters from phage λ, a glycolytic yeast promoter, a promoter from the yeast acid phosphatase gene, a yeast alpha-mating factor, and promoters derived from adenovirus, Epstein-Barr virus, polyoma, and simian virus, as well as those from various retroviruses. However, numerous other vectors and control elements suitable for procaryotic or eucaryotic expression are known in the art and may be employed in the practice of this invention.

Examples of suitable procaryotic cloning vectors may include plasmids from *E. coli* (e.g. pBR322, col E1, pUC, and the F-factor), with preferred plasmids being pCFM1156 (ATCC 69702), pCFM1656 (ATCC 69576) and pCFM3102 (described in the Examples section, below). Other appropriate expression vectors of which numerous types are known in the art for mammalian, insect, yeast, fungal and bacterial expression can also be used for this purpose. The transfection of these vectors into appropriate host cells can result in expression of the KGF analog polypeptides.

Host microorganisms useful in this invention may be either procaryotic or eucaryotic. Suitable procaryotic hosts include various *E. coli* (e.g., FM5, HB101, DH5α,DH10, and MC1061), *Pseudomonas, Bacillus,* and *Streptomyces* strains, with *E. coli* being preferred. Suitable eucaryotic host cells include yeast and other fungi, insect cells, plant cells, and animal cells, such as COS (e.g., COS-1 and COS-7) and CV-1 monkey cell lines, 3T3 lines derived from Swiss, Balb-c or NIH cells, HeLa and L-929 mouse cells, and CHO, BHK or HaK hamster cells. Depending upon the host employed, recombinant polypeptides produced in accordance herewith will be glycosylated with mammalian or other eucaryotic carbohydrates or may be non-glycosylated.

The preferred production method will vary depending upon many factors and considerations; the optimum production procedure for a given situation will be apparent to those skilled in the art through minimal experimentation. The resulting expression product may then be purified to near homogeneity using procedures known in the art. A typical purification procedure for procaryotic cell production involves rupturing the cell walls by high pressure or other means, centrifugation or filtration to remove cellular debris, followed by ion exchange chromatography of supernatant or filtrate and, finally, hydrophobic interaction chromatography. If the analog is expressed in insoluble form, another purification technique involves first solublizing the inclusion bodies containing the analogs followed by ion exchange chromatography, then refolding of the protein, and, finally, hydrophobic interaction chromatography. Exemplary purification techniques are taught in commonly owned U.S.S.N. 08/323,339, filed on October 13, 1994. Generally, U.S.S.N. 08/323,339 teaches a method for purifying a keratinocyte growth factor comprising: (a) obtaining a solution comprising the KGF; (b) binding the KGF from the solution of part (a) to a cation exchange resin; (c) eluting the KGF in an eluate solution from the cation exchange resin; (d) either passing the eluate solution from part (c) through an appropriate molecular weight exclusion matrix or performing hydrophobic interaction chromatography on the eluate solution of part (c); and (e) recovering the KGF from the molecular weight exclusion matrix or hydrophobic interaction chromatography.

Of course, the analogs may be rapidly screened to assess their physical properties. The Examples sets forth various well-known stability assays, although the specific assay used to test the analog is not critical. Moreover, the level of biological activity (e.g., receptor binding and/or affinity, mitogenic, cell proliferative and/or *in vivo* activity) may also be tested using a variety of assays, some of which are set forth in the Examples section. Numerous assays are well-known and can be used to quickly screen the KGF analogs to determine whether or not they possess acceptable biological activity. One such assay specifically tests the KGF analogs for the ability to bind to the KGF receptor (KGFR) by competing with ¹²⁵I-KGF binding (Bottaro *et al.* (1990), *J. Biol. Chem.,* 265:12767-12770; Ron *et al*. (1993), *J. Biol. Chem.,* 268:2984-2988). An alternative method for assaying KGFR/KGF analog interactions involves the use of techniques such as real time biospecific interaction analysis (BIA) (Felder *et al*. (1993), *Molecular & Cellular Biology,* 13:1449-1455). Additionally a mitogenic assay can be utilized to test the ability of the KGF analogs to stimulate DNA synthesis (Rubin *et al.* (1989), *supra).* Finally, cell proliferative assays can be utilized to test the ability of the KGF analogs to stimulate cell proliferation (Falco, *et al.* (1988), *Oncogene,* 2:573-578). Using any of the aforementioned assay systems, KGF analogs can be rapidly screened for their biological activity.

The KGF analogs may be further modified to contain additional chemical moieties not normally a part of the peptide. Such derivatized moieties may improve the solubility, absorption, biological half life, and the like of the KGF analog. The moieties may alternatively eliminate or attenuate any undesirable side effects of the protein and the like. Moieties capable of mediating such effects are disclosed, for example, in *REMINGTON'S PHARMACEUTICAL SCIENCES,* 18th ed., Mack Publishing Co., Easton, PA (1990). Covalent modifications may be introduced into the molecule by reacting targeted amino acid residues of the peptide with an organic derivatizing agent that is capable of reacting with selected side chains or terminal residues (T.E. Creighton (1983), *PROTEINS: STRUCTURE AND MOLECULE PROPERTIES,* W.H. Freeman & Co., San Francisco, pp. 79-86). Polyethylene glycol ("PEG") is one such chemical moiety which has been used in the preparation of therapeutic protein products. For some proteins, the attachment of polyethylene glycol has been shown to protect against proteolysis, Sada, *et al.* (1991), *J. Fermentation Bioengineering,* 71:137-139 , and methods for attachment of certain polyethylene glycol moieties are available. See U.S. Patent No. 4,179,337, Davis *et al*., "Non-Immunogenic Polypeptides," issued December 18, 1979; and U.S. Patent No. 4,002,531, Royer, "Modifying enzymes with Polyethylene Glycol and Product Produced Thereby," issued January 11, 1977. For a review, see Abuchowski *et al.*, in Enzymes as Drugs. (Holcerberg and Roberts, (eds.) pp. 367-383 (1981)). For polyethylene glycol, a variety of means have been used to attach the polyethylene glycol molecules to the protein. Generally, polyethylene glycol molecules are connected to the protein via a reactive group found on the protein. Amino groups, such as those on lysine residues or at the N-terminus, are convenient for such attachment. For example, Royer (U.S. Pat. No. 4,002,531, above) states that reductive alkylation was used for attachment of polyethylene glycol molecules to an enzyme. EP 0 539 167, published April 28, 1993, Wright, "PEG Imidates and Protein Derivates Thereof" states that peptides and organic compounds with free amino group(s) are modified with an imidate derivative of PEG or related water-soluble organic polymers. U.S. Patent No. 4,904,584, Shaw, issued February 27, 1990, relates to the modification of the number of lysine residues in proteins for the attachment of polyethylene glycol molecules via reactive amine groups.

In yet another embodiment, the present invention is directed to a single-dose administration unit of a medicinal formulation which can be safely administered parenterally or orally to treat a disease in a warm-blooded animal (such as a human). Such medicinal formulation may be in the form of a lyophilized or otherwise dehydrated therapeutic or diagnostic which can be reconstituted by the addition of a physiologically acceptable solvent. The solvent may be any media such as sterile water, physiological saline solution, glucose solution or other aqueous carbohydrates (e.g., polyols such as mannitol, xylitol, glycerol) which is capable of dissolving the dried composition, is compatible with the selected administration route and which does not negatively interfere with the active principle and the reconstitution stabilizers employed. In a specific embodiment, the present invention is directed to a kit for producing the single-dose administration unit. The kit contains both a first container having a dried protein and a second container having an aqueous formulation comprising a reconstitution stabilizer. As for the concentration of the protein in the solution, the solution volume which is charged into each container, and the capacity of the containers (interrelated parameters which can be suitably modified, depending upon the desired concentration of active principle in the end-dosage unit), these may vary within wide ranges well-known to skilled artisans.

KGF analogs according to the invention may be useful as therapeutic and diagnostic agents and as research reagents. Thus the KGF analogs may be used in *in vitro* and/or *in vivo* diagnostic assays to quantify the amount of KGF in a tissue or organ sample or to determine and/or isolate cells which express KGFR (Bottaro *et al.* (1990), *J. Biol. Chem.,* 265:12767-12770; Ron *et al.* (1993), *J. Biol. Chem.,* 268:2984-2988). In assays of tissues or organs there will be less radioactivity from ¹²⁵I-KGF analog binding to KGFR, as compared to a standardized binding curve of ¹²⁵I-KGF analog, due to unlabeled native KGF binding to KGFR. Similary, the use of ¹²⁵I-KGF analog may be used to detect the presence of KGFR in various cell types.

This invention also contemplates the use of a KGF analog in the generation of antibodies made against the peptide, which antibodies also bind to native KGF. In this embodiment, the antibodies are monoclonal or polyclonal in origin and are generated using a KGF analog. The resulting antibodies bind preferentially to native KGF, preferably when that protein is in its native (biologically active) conformation. These antibodies can be used for detection or purification of the KGF.

Moreover, the invention contemplates the use of KGF analogs in the discovery of high affinity or low affinity KGF binding molecules having therapeutical applications, for example, as a way for efficient KGF delivery or as an inhibitor for KGF activity. The thermal stability of the KGF analogs is important to identify such binding molecules in physiological conditions (i.e., at 37°C) since their affinity for KGF could be strongly temperature-dependent and may be unpredictable from the affinity observed at 4°C.

For *in vivo* uses, the KGF analogs may be formulated with additives. Such additives include buffers, carriers, stabilizers, excipients, preservatives, tonicity adjusting agents, anti-oxidants and the like (*e.g*., viscosity adjusting agents or extenders). The selection of specific additives will depend upon the storage form (i.e., liquid or lyophilized) and the modes of administering the KGF analog. Suitable formulations, known in the art, can be found in *REMINGTON'S PHARMACEUTICAL SCIENCES* (latest edition), Mack Publishing Company, Easton, PA.

The KGF analogs may be applied in therapeutically effective amounts to tissues specifically characterized by having damage to or clinically insufficient numbers of non-fibroblast epithelium cells. Since KGF binds to heparin, it is likely that heparin, heparin sulfate, heparin-like glycosaminglycans and heparin-like glycosaminoglycans, which are present in the extracellular environment may bind KGF *in vivo*. It follows that KGF analogs with reduced heparin binding ability will have enhanced potency, as more KGF will reach its targeted receptor and will not be sequestered by heparin and heparin-like compounds in the extracellular environment. These analogs will be more useful therapeutically, as lower dosages of a particular KGF analog will be required per treatment.

The KGF analogs may be applied in therapeutically effective amounts to tissues specifically characterized by having damage to or clinically insufficient numbers of non-fibroblast epithelium cells. Areas in which KGF analogs may be successfully administered include, but are not limited to: the stimulation, proliferation and differentiation of adnexal structures such as hair follicles, sweat glands, and sebaceous glands in patients with burns and other partial and full-thickness injuries; accelerated reepithelialization of lesions caused by epidermolysis bullosa, which is a defect in adherence of the epidermis to the underlying dermis, resulting in frequent open, painful blisters which can cause severe morbidity; preventing chemotherapy-induced alopecia and treating male-pattern baldness, or the progressive loss of hair in men and women; treating gastric and duodenal ulcers; treating inflammatory bowel diseases, such a Crohn's disease (affecting primarily the small intestine) and ulcerative colitis (affecting primarily the large bowel); preventing or reducing gut toxicity in radiation and chemotherapy treatment regimes through treatment (e.g., pretreatment and/or postreatment) to induce a cytoprotective effect or regeneration or both; stimulating the production of mucus throughout the gastrointestinal tract; inducing the proliferation and differentiation of type II pneumocytes, which may help treat or prevent diseases such as hyaline membrane disease (i.e., infant respiratory distress syndrome and bronchopulmonary dysplasia) in premature infants; stimulating the proliferation and differentiation of the bronchiolar and/or alveolar epithelium with acute or chronic lung damage or insufficiency due to inhalation injuries (including high oxygen levels), emphysema, use of lung damaging chemotherapeutics, ventilator trauma or other lung damaging circumstances; increasing liver function to treat or prevent hepatic cirrhosis, fulminant liver failure, damage caused by acute viral hepatitis and/or toxic insults to the liver; inducing corneal cell regeneration, for example in the treatment of corneal abrasion; inducing epithelial cell regeneration to treat progressive gum disease; inducing regeneration of tympanic epithelial cells to treat ear drum damage and treating or preventing the onset of diabetes mellitus or as an adjunct in the setting of islet cell transplantation.

A patient in need of proliferation of non-fibroblast epithelial cells will be administered an effective amount of a KGF analog. An "effective amount" is that amount of KGF analog required to elicit the desired response in the patient being treated and will, thus, generally be determined by the attending physician. Factors influencing the amount of KGF analog administered will include the age and general condition of the patient, the disease being treated, etc. Typical dosages will range from 0.001 mg/kg body weight to 500 mg/kg body weight.

The KGF analog may be safely administered parenterally *(e.g.,* via IV, IT, IM, SC, or IP routes), orally or topically to warm-blooded animals (such as humans). The KGF analog may be used once or administered repeatedly, depending on the disease and condition of the patient. In some cases, the KGF analog may be administered as an adjunct to other therapy and also with other pharmaceutical preparations.

The following examples are included to more fully illustrate the present invention.

### EXAMPLES

Standard methods for many of the procedures described in the following examples, or suitable alternative procedures, are provided in widely recognized manuals of molecular biology such as, for example, *Molecular Cloning,* Second Edition, Sambrook *et al.,* Cold Spring Harbor Laboratory Press (1987) and *Current Protocols in Molecular Biology,* Ausabel *et al.,* Greene Publishing Associates/Wiley Interscience, New York (1990).

### EXAMPLE 1: Preparation of DNA Coding for KGF and KGF Analogs

The cloning of the full-length human KGF gene (encoding a polypeptide with the sequence of native KGF) was carried out both by polymerase chain reaction (PCR) of RNA from an animal cell and by PCR of chemically synthesized (*E.* coli optimized codon) oligonucleotides ("OLIGOs"). Both procedures are described below:

PCR amplification using RNA isolated from cells known to produce the polypeptide was performed. Initially, cells from a human fibroblast cell line AG1523A (obtained from Human Genetic Mutant Cell Culture Repository Institute For Medical Research, Camden, New Jersey) were disrupted with guanidium thiocyanate, followed by extraction (according to the method of Chomyzinski *et al.* (1987), *Anal. Biochem.,* 172:156). Using a standard reverse transcriptase protocol for total RNA, the KGF cDNA was generated. PCR (PCR#1) amplification of the KGF gene was carried out using the KGF cDNA as template and primers OLIGO#1 and OLIGO#2 that encode DNA sequences immediately 5' and 3' of the KGF gene [Model 9600 thermocycler (Perkin-Elmer Cetus, Norwalk, CT); 28 cycles; each cycle consisting of one minute at 94°C for denaturation, two minutes at 60°C for annealing, and three minutes at 72°C for elongation]. A small aliquot of the PCR#1 product was then used as template for a second KGF PCR (PCR#2) amplification identical to the cycle conditions described above except for a 50°C annealing temperature. For expression cloning of the KGF gene, nested PCR primers were used to create convenient restriction sites at both ends of the KGF gene. OLIGO#3 and OLIGO#4 were used to modify the KGF DNA product from PCR#2 to include *MluI* and *BamHI* restriction sites at the 5' and 3' ends of the gene, respectively [PCR#3; 30 cycles; each cycle consisting of one minute at 94°C for denaturation, two minutes at 60°C for annealing, and three minutes at 72°C for elongation]. This DNA was subsequently cut with *MluI* and *BamHI*, phenol extracted, and ethanol precipitated. It was then resuspended and ligated (using T4 ligase) into a pCFM1156 plasmid (Figure 2A) that contained a "RSH" signal sequence to make construct RSH-KGF (Figure 3).

The ligation products were transformed (according to the method of Hanahan (1983), *J. Mol. Biol.,* 166:557) into *E. coli* strain FM5 (ATCC: 53911) and plated onto LB+kanamycin at 28°C. Several transformants were selected and grown in small liquid cultures containing 20 µg/mL kanamycin. The RSH-KGF plasmid was isolated from the cells of each culture and DNA sequenced. Because of an internal *NdeI* site in the KGF gene, it was not possible to directly clone the native gene sequence into the desired expression vector with the bracketed restriction sites of *NdeI* and *BamHI.* This was accomplished as a three-way ligation. Plasmid RSH-KGF was cut with the unique restriction sites of *BsmI* and *SstI*, and a ∼3 kbp DNA fragment (containing the 3' end of the KGF gene) was isolated following electrophoresis through a 1% agarose gel. A PCR (PCR#4) was carried out as described for PCR#3 except for the substitution of OLIGO#5 for OLIGO#3. The PCR DNA product was then cut with *NdeI* and *BsmI* and a 311 bp DNA fragment was isolated following electrophoresis through a 4% agarose gel. The third piece of the ligation is a 1.8 kbp DNA fragment of pCFM1156 cut with *NdeI* and *SstI* which was isolated following electrophoresis through a 1% agarose gel. Following ligation (T4 ligase), transformation, kanamycin selection and DNA sequencing as described above, a clone was picked containing the construct in Figure 4 and the plasmid designated KGF. Because of an internal ribosomal binding site that produced truncated products, the KGF DNA sequence between the unique *KpnI* and *EcoRI* sites was replaced with chemically synthesized OLIGOs (OLIGO#6 through OLIGO#11) to minimize the use of the internal start site (Figure 5).

OLIGO#1 (SEQ ID NO:7):     5'-CAATGACCTAGGAGTAACAATCAAC-3'

OLIGO#2 (SEQ ID NO:8):     5'-AAAACAAACATAAATGCACAAGTCCA-3'

OLIGO#3 (SEQ ID NO:9):     5'-ACAACGCGTGCAATGACATGACTCCA-3'

OLIGO#4 (SEQ ID NO:10):
5'-ACAGGATCCTATTAAGTTATTGCCATAGGAA-3'

OLIGO#5 (SEQ ID NO:11):
5'-ACACATATGTGCAATGACATGACTCCA-3'

OLIGO#6 (SEQ ID NO:12):
5'-CTGCGTATCGACAAACGCGGCAAAGTCAAGGGCACCC-3'

OLIGO#7 (SEQ ID NO:13):
5'-AAGAGATGAAAAACAACTACAATATTATGGAAATCCGTACTGTT-3'

OLIGO#8 (SEQ ID NO:14):
5'-GCTGTTGGTATCGTTGCAATCAAAGGTGTTGAATCTG-3'

OLIGO#9 (SEQ ID NO:15):
5'-TCTTGGGTGCCCTTGACTTTGCCGCGTTTGTCGATACGCAGGTAC-3'

OLIGO#10 (SEQ ID NO:16):
5'-ACAGCAACAGTACGGATTTCCATAATATTGTAGTTGTTTTTCATC-3'

OLIGO#11 (SEQ ID NO:17):
5'-AATTCAGATTCAACACCTTTGATTGCAACGATACCA-3'

The OLIGOs were phosphorylated with T4 polynucleotide kinase and then heat denatured. The single-stranded (ss) OLIGOs were then allowed to form a ds DNA fragment by allowing the temperature to slowly decrease to room temperature. T4 ligase was then used to covalently link both the internal OLIGO sticky-ends and the whole ds OLIGO fragment to the KGF plasmid cut with *KpnI* and *EcoRI.* The new plasmid was designated KGF(dsd).

A completely *E. coli* codon-optimized KGF gene was constructed by PCR amplification of chemically synthesized OLIGOs #12 through 24.

OLIGO#12 (SEQ ID NO:18): 5'-AGTTTTGATCTAGAAGGAGG-3'

OLIGO#13 (SEQ ID NO:19): 5'-TCAAAACTGGATCCTATTAA-3'

OLIGO#14 (SEQ ID NO:20):
5'-AGTTTTGATCTAGAAGGAGGAATAACATATGTGCAACGACATG-
ACTCCGGAACAGATGGCTACCAACGTTAACTGCTCCAGCCCGGAACGT-3'

OLIGO#15 (SEQ ID NO:21):
5'-CACACCCGTAGCTACGACTACATGGAAGGTGGTGACATCCGT-
GTTCGTCGTCTGTTCTGCCGTACCCAGTGGTACCTGCGTATCGACAAA-3'

OLIGO#16 (SEQ ID NO:22):
5'-CGTGGTAAAGTTAAAGGTACCCAGGAAATGAAAAACAACTACAACATC-
ATGGAAATCCGTACTGTTGCTGTTGGTATCGTTGCAATCAAA-3'

OLIGO#17 (SEQ ID NO:23):
5'-GGTGTTGAATCTGAATTCTACCTGGCAATGAACAAAGAAGGTAAACT-
GTACGCAAAAAAAGAATGCAACGAAGACTGCAACTTCAAAGAA-3'

OLIGO#18 (SEQ ID NO:24):
5'-CTGATCCTGGAAAACCACTACAACACCTACGCATCTGCTAAATGGAC-
CCACAACGGTGGTGAAATGTTCGTTGCTCTGAACCAGAAAGGT-3'

OLIGO#19 (SEQ ID NO:25):
5'-ATCCCGGTTCGTGGTAAAAAAACCAAAAAAGAACAGAAAACCGCTC-
ACTTCCTGCCGATGGCAATCACTTAATAGGATCCAGTTTTGA-3'

OLIGO#20 (SEQ ID NO:26):5'-TACGGGTGTGACGTTCCGGG-3'

OLIGO#21 (SEQ ID NO:27):5'-CTTTACCACGTTTGTCGATA-3'

OLIGO#22 (SEQ ID NO:28):5'-ATTCAACACCTTTGATTGCA-3'

OLIGO#23 (SEQ ID NO:29):5'-CCAGGATCAGTTCTTTGAAG-3'

OLIGO#24 (SEQ ID NO:30):5'-GAACCGGGATACCTTTCTGG-3'

OLIGOs #12 through 24 were designed so that the entire DNA sequence encoding native KGF was represented by OLIGOs from either the "Watson" or the "Crick" strand and upon PCR amplification would produce the desired double-stranded DNA sequence (Figure 6) [PCR#5, Model 9600 thermocycler, Perkin-Elmer Cetus]; 21 cycles, each cycle consisting of 31 seconds at 94°C for denaturation, 31 seconds at 50°C for annealing, and 31 seconds at 73°C for elongation; following the 21 cycles the PCR was finished with a final elongation step of 7 minutes]. After PCR amplification, the DNA fragment was cut with *XbaI* and *BamHI* and the 521 bp fragment ligated into the expression plasmid pCFM1156 cut with the same enzymes. PCR#5 utilized the outside primers (100 pmoles/100 µl rxn) OLIGO#12 and OLIGO#13 and 1 µl/100 µl rxn of a KGF template derived by ligation (by T4 ligase) of OLIGO #14 through OLIGO#19 (OLIGO#15 through OLIGO#18 were phosphorylated with T4 polynucleotide kinase) using OLIGO#20 through OLIGO#24 as band-aid oligos (Jayaraman *et al.* (1992), *Biotechniques,* 12:392) for the ligation. The final construct was designated KGF (codon optimized).

All of the KGF analogs described herein are composed in part from DNA sequences found in KGF(dsd) or KGF(codon optimized), or a combination of the two. The sequences are further modified by the insertion into convenient restriction sites of DNA sequences that encode the particular KGF analog amino acids made utilizing one or more of the above-described techniques for DNA fragment synthesis. Any of the analogs can be generated in their entirety by the above described techniques. However, as a part of the general OLIGO design optimized *E. coli* codons were used where appropriate, although the presence of *E. coli* optimized codons in part or *in toto* of any of the genes where examined did not significantly increase the yield of protein that could be obtained from cultured bacterial cells. Figures 7 to 10 and 17 to 26 set forth by convenient example particular KGF analog nucleotide and amino acid sequence constructions: R(144)Q (Figure 7); C(1,15)S/R(144)E (Figure 8); C(1,15)S/R(144)Q (Figure 9); ΔN23/R(144)Q (Figure 10); ΔN23/N(137)E (Figure 17); ΔN23/K(139)E (Figure 18); ΔN23/K(139)Q (Figure 19); ΔN23/R(144)A (Figure 20); ΔN23/R(144)L (Figure 21); ΔN23/K(147)E (Figure 22); ΔN23/K(147)Q (Figure 23); ΔN23/K(153)E (Figure 24); ΔN23/K(153)Q; (Figure 25) and ΔN23/Q(152)E/K(153)E (Figure 26). All the KGF analog constructions described herein were DNA sequence confirmed.

### EXAMPLE 2: Production in E. coli

Three different expression plasmids were utilized in the cloning of the KGF analog genes. They were pCFM1156 (ATCC# 69702), pCFM1656 (ATCC# 69576), and pCFM3102 (Figures 2A, 2B and 2C, respectively). The plasmid p3102 can be derived from the plasmid pCFM1656 by making a series of site-directed base changes with PCR overlapping oligo mutagenesis. Starting with the *BglII* site (pCFM1656 plasmid bp # 180) immediately 5' to the plasmid replication promoter, PcopB, and proceeding toward the plasmid replication genes, the base pair changes are as follows:

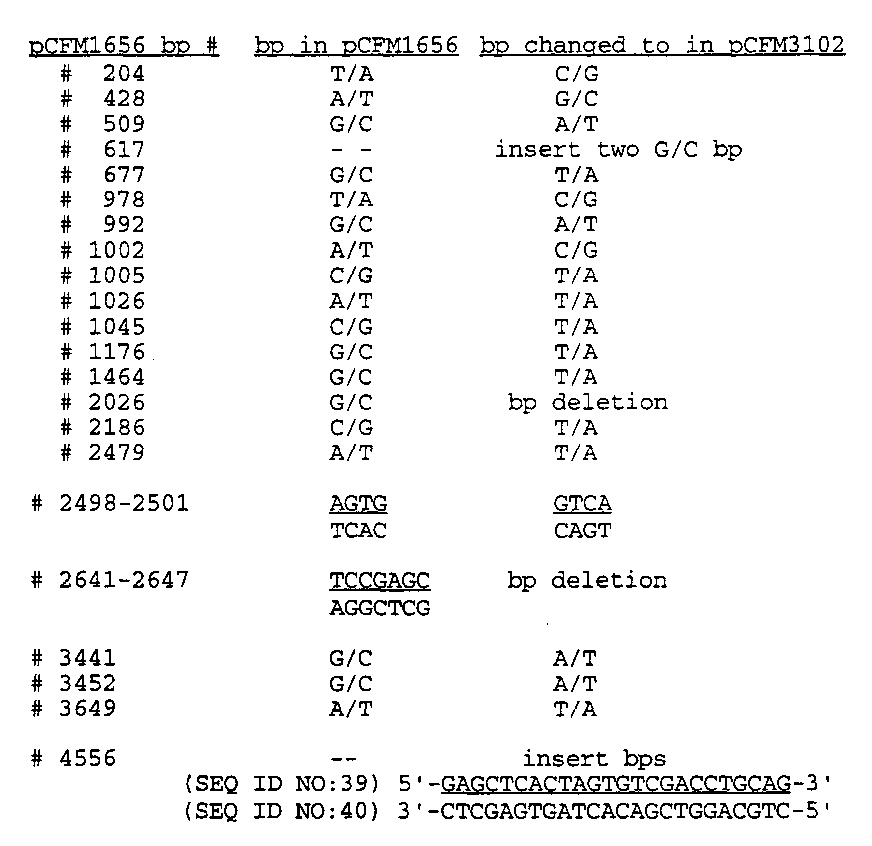

As seen above, pCFM1156, pCFM1656 and pCFM3102 are very similar to each other and contain many of the same restriction sites. The plasmids were chosen by convenience, and the vector DNA components can be easily exchanged for purposes of new constructs. The host used for all cloning was *E. coli* strain FM5 (ATCC: 53911) and the transformations were carried out (according to the method of Hanahan (1983), *supra)* or by electroelution with a Gene Pulser™ transfection apparatus (BioRad Laboratories, Inc., Hercules, CA) according to the manufacturer's instructions.

Initially, a small, freshly cultured inoculum of the desired recombinant *E. coli* clone harboring the desired construct on one of the three pCFM vectors was started by transferring 0.1 mL of a frozen glycerol stock of the appropriate strain into a 2 L flask containing 500 mL of Luria broth. The culture was shaken at 30°C for 16 hours, after which the culture was transferred to a 15 L fermentor containing 8 L of sterile batch medium (Tsai, *et al.* (1987), *J. Industrial Microbiol.,* 2:181-187).

Feed batch fermentation starts with the feeding of Feed # 1 medium (Tsai, *et al.* (1987), *supra).* When the OD600 reached 35, expression of the desired KGF analog was induced by rapidly raising the culture temperature to 37°C for two hours then up to 42°C to denature the CI repressor. The addition of Feed 1 was discontinued in favor of Feed 2, the addition rate of which was initiated at 300 mL/hr. Feed 2 comprised 175 g/L trypticase-peptone, 87.5 g/L yeast extract, and 260 g/L glucose. After one hour at 42°C, the culture temperature was decreased to 36°C, where this temperature was then maintained for another 6 hours.

The fermentation was then halted and the cells were harvested by centrifugation into plastic bags placed within 1 L centrifuge bottles. The cells were pelleted by centrifugation at 400 rpm for 60 minutes, after which the supernatants were removed and the cell paste frozen at -90°C.

Following expression of the various KGF analogs, in *E. coli,* native KGF, R(144)Q, C(1,15)S/R(144)E, C(1,15)S/R(144)Q and ΔN23/R(144)Q proteins were purified using the following procedure. Cell paste from a high cell density fermentation was suspended at 4°C in 0.2 M NaCl, 20 mM NaPO₄, pH 7.5 as a 10-20% solution (weight per volume) using a suitable high shear mixer. The suspended cells were then lysed by passing the solution through a homogenizer (APV Gaulin, Inc., Everett, MA) three times. The outflowing homogenate was cooled to 4-8°C by using a suitable heat exchanger. Debris was then removed by centrifuging the lysate in a J-6B™ centrifuge (Beckman Instruments, Inc., Brea, CA) equipped with a JS 4.2 rotor at 4,200 rpm for 30-60 min. at 4°C. Supernatants were then carefully decanted and loaded onto a previously prepared 450 mL (5 cm x 23 cm) column of S-Sepharose Fast Flow™ resin (Pharmacia, Piscataway, NJ) equilibrated with 0.2 M NaCl, 20 mM NaPO₄, pH 7.5 at 4°C. Next, the column was washed with five column volumes (2250 mL) of 0.4 M NaCl, 20 mM NaPO₄, pH 7.5 at 4°C. The desired protein was eluted by washing the column with 5 L of 0.5 M NaCl, 20 mM NaPO₄, pH 7.5. Then, 50 mL fractions were collected and the A₂₈₀ of the effluent was continuously monitored. Fractions identified by A₂₈₀ as containing eluted material were then analyzed by SDS-PAGE through 14% gels to confirm the presence of the desired polypeptide.

Those fractions containing proteins of interest were then pooled, followed by the addition of an equal volume of distilled water. The diluted sample was then loaded onto a previously prepared 450 mL (5 cm x 23 cm) column of S-Sepharose Fast Flow equilibrated with 0.4 M NaCl 20 mM NaPO₄, pH 6.8 at 4°C. The column was washed with 2250 mL of 0.4 M NaCl, 20 mM NaPO₄, pH 6.8 and the protein eluted using a 20 column volume linear gradient ranging from 0.4 M NaCl, 20 mM NaPO₄, pH 6.8 to 0.6 M NaCl, 20 mM NaPO₄, pH 6.8. Again, 50 mL fractions were collected under constant A₂₈₀ monitoring of the effluent. Those fractions containing the protein (determined by 14% SDS-PAGE) were then pooled, followed by concentration through a YM-10 membrane (10,000 molecular weight cutoff) in a 350cc stirring cell (Amicon, Inc. Mayberry, MA) to a volume of 30-40 mL.

The concentrate was then loaded onto a previously generated 1,300 mL (4.4 cm x 85 cm) column of Superdex-75™ resin (Pharmacia) equilibrated in column buffer comprising 1X PBS (Dulbecco's Phosphate Buffered Saline, "D-PBS", calcium and magnesium-free) or 0.15 M NaCl, 20 mM NaPO₄, pH 7.0. After allowing the sample to run into the column, the protein was eluted from the gel filtration matrix using column buffer. Thereafter, 10 mL fractions were recovered and those containing the analog (determined by 14% SDS-PAGE) were pooled. Typically, the protein concentration was about 5-10 mg/mL in the resultant pool. All of the above procedures were performed at 4-8°C, unless otherwise specified.

### Analysis

Analysis was conducted on *E. coli*-derived, native KGF, R(144)Q; C(1,15)S/R(144)E; C(1,15)S/R(144)Q and ΔN23/R(144)Q.

### Conformational Stability

The polypeptides were compared by their storage stability, thermal unfolding transition temperatures (Tₘ), and stability in a broad range of pH conditions.

The ability of native KGF, C(1,15)S, R(144)Q,C(1,15)S/R(144)Q, C(1,15)S/R(144)E and ΔN23/R(144)Q to prevent aggregation at elevated temperatures was also examined. Samples containing 0.5 mg/mL of protein were prepared in D-PBS. 0.5 mL of each sample was aliquoted into 3 cc type-1 glass vials. The vials were sealed with rubber stoppers and 13 mm flip-off aluminum seals were crimped on. These vials were then placed in a 37°C incubator. At predetermined time intervals, vials were withdrawn and analyzed for the loss of soluble protein. Visible precipitates were removed by centrifuging 250 µL of each sample through a 0.22 µm Spin-X filter unit (Costar, Cambridge, MA). Soluble protein in the filtered solutions was subsequently analyzed by size exclusion HPLC. The amount of soluble protein was determined by integrating the HPLC peak area and plotting the result as a function of incubation time at 37°C. The results of native KGF, C(1,15)S, C(1,15)S/R(144)Q, and C(1, 15)S/R(144)E are shown in Figure 11. The data for R(144)Q and ΔN23/E(144)Q are not shown.

The half-lives for the loss of soluble, monomeric protein were then estimated from these kinetic curves. Table 1 shows the half-life for remaining soluble KGF upon storage at 37°C for these proteins.

**Table 1**

| Half-life for the Loss of Soluble, Monomeric Proteins | |
|---|---|
| Protein | t1/2 (day) |
| native KGF | 0.6 |
| R(144)Q | 4.1 |
| C(1,15)S/R(144)Q | 13.3 |
| ΔN23/R(144)Q | 22.3 |
| C(1,15)S/R(144)E | 38.0 |

As seen in Table 1, above, and Figure 11, the native KGF aggregated the most rapidly, with a half-life of 0.6 days. R(144)Q increased the half-life to 4.1 days. C(1,15)S/R(144)Q, ΔN23/R(144)Q and C(1,15)S/R(144)E showed substantial increases in the solubility half-life to 13.3, 22.3 and 38 days, respectively.

### Thermal Unfolding

Thermal unfolding was monitored by circular dichroism (CD) at 230 nm using a J-720™ spectropolarimeter (Jasco, Inc., Easton, MD) equipped with a PTC-343 Peltier-type temperature control system. For CD analysis, separate samples containing 0.1 mg/mL of the polypeptide to be analyzed were prepared in D-PBS (Life Technologies, Inc., Grand Island, NY). For each sample, about 2.5 mL was loaded into a 10 mm path length rectangular Suprasil™ quartz (Heraeus Quarzschmelze, GmbH, Hanau, Germany) fluorescent cell (Hellma Cells, Inc., Jamaica, NY). The cell was then placed into the Peltier-type temperature control system in the spectropolarimeter. Thermal unfolding was carried out at a rate of 50°C/hr. Changes in ellipticity were monitored at 230 nm to indicate unfolding. The Tₘ of each sample was estimated by identifying a temperature at which 50% of protein molecules in the solution were unfolded (*Biophysical Chemistry,* Cantor and Schimmel (eds), W.H. Freeman and Co. San Francisco (1980)). The estimated Tₘ for each of the five proteins is listed in Table 2.

**Table 2**

| Estimated Melting Temperatures | |
|---|---|
| Protein | Tₘ (°C) |
| native KGF | 54.0 |
| R(144)Q | 61.5 |
| C(1,15)S/R(144)Q | 62.5 |
| ΔN23/R(144)Q | 63.0 |
| C(1,15)S/R(144)E | 63.5 |

As the results show, R(144)Q has a greater than 7°C increase in the Tm as compared with native KGF. The substitution of R(144)Q to C(1,15)S/R(144)Q or ΔN23 adds at least another 1°C increase in Tₘ and more than 8°C as compared with native KGF. Moreover, the C(1,15)S/R(144)E is greater than 9°C more stable than native KGF. Therefore, switching a positively charged residue (Arg) at amino acid position 144 to a neutrally or negatively charged residue substantially stabilized the polypeptide.

### pH

The acid stabilities of C(1,15)S/R(144)Q, C(1,15)S, and C(1,15)S/R(144)E were also compared to that of native KGF, by adjusting D-PBS to different pH values by adding concentrated HCl or NaOH. Approximately 2.35 mL of D-PBS at different pH values was mixed with 100 µL of 2.45 mg/mL KGF protein in a quartz cell. These samples were thermally unfolded at a rate of 50°C/hr and monitored by CD at 230 nm. Figure 12 shows the Tₘ as a function of pH for native KGF, C(1,15)S/R(144)Q and C(1,15)S/R(144)E. In the pH range tested, the C(1,15)S/R(144)Q and C(1,15)S/R(144)E always have a higher Tₘ than the native KGF.

### In vitro Biological Activity

*In vitro* mitogenic activities of R(144)Q, ΔN23/R(144)Q, C(1,15)S/R(144)Q,C(1,15)S, and C(1,15)S/R(144) E were also determined as a function of protein concentration and the half-maximal concentrations by measurement of [³H]-thymidine uptake by Balb/MK cells (according to the methods of Rubin *et al*. (1989), *supra).*

Generally, the concentrations of each of the KGF analogs relative to a known standard native KGF was determined using an *in vitro* biological assay. Each KGF analog was then diluted and assayed for biological activity using a Balb/MK mitogenic assay. The samples were first diluted in a bioassay medium consisting of 50% customer-made Eagle's MEM, 50% customer-made F12, 5 µG/mL transferrin, 5 ng/ml sodium selenite, 0.0005% HSA and 0.005% Tween 20. KGF samples were then added into Falcon Primeria 96-well plates seeded with Balb/MK cells. Incorporation of [³H]-Thymidine during DNA synthesis was measured and converted to input native KGF concentration by comparison to a native KGF standard curve. The results are presented in Figures 13 to 16. As seen in Figures 13 to 16, each of the KGF analogs has mitogenic activity.

## Claims

1. A polypeptide analog of native keratinocyte growth factor termed "KGF," wherein native KGF corresponds to the following sequence 
Cys
1

Asn Asp Met Thr Pro Glu Gln Met Ala Thr Asn Val Asn Cys Ser Ser
            5                   10                  15

Pro Glu Arg His Thr Arg Ser Tyr Asp Tyr Met Glu Gly Gly Asp Ile
        20                  25                  30

Arg Val Arg Arg Leu Phe Cys Arg Thr Gln Trp Tyr Leu Arg Ile Asp
    35                  40                  45

Lys Arg Gly Lys Val Lys Gly Thr Gln Glu Met Lys Asn Asn Tyr Asn
50 50 60 65

Ile Met Glu Ile Arg Thr Val Ala Val Gly Ile Val Ala Ile Lys Gly
                70                  75                  80

Val Glu Ser Glu Phe Tyr Leu Ala Met Asn Lys Glu Gly Lys Leu Tyr
            85                  90                  95

Ala Lys Lys Glu Cys Asn Glu Asp Cys Asn Phe Lys Glu Leu Ile Leu
        100                 105                 110

Glu Asn His Tyr Asn Thr Tyr Ala Ser Ala Lys Trp Thr His Asn Gly
    115                 120                 125

Gly Glu Met Phe Val Ala Leu Asn Gln Lys Gly Ile Pro Val Arg Gly
130                 135                 140                 145

Lys Lys Thr Lys Lys Glu Gln Lys Thr Ala His Phe Leu Pro Met Ala
                150                 155                 160

Ile Thr
 said analog having an improved stability wherein the analog comprises the amino acid sequence of KGF having a charge-change by the deletion and/or substitution of one or more of amino acid residues 41-154 of the above sequence to effect a reduced positive charge as compared with native KGF, optionally further having a deletion of the first 15 to 24 amino acids of the N-teminal native KGF, having residues corresponding to Cys (1) and Cys (15) replaced or deleted, having an N-terminal methionine or having a signal sequence, with the proviso that the amino acid sequence 123-131 of the above sequence is not substituted by any one of the amino acid sequences selected from the group of DLYQG and AKYEG.

2. The polypeptide analog according to claim 1 wherein the deleted or substituted amino acid residues are selected form the arginine residue at amino acid position 41, glutamine residue at amino acid position 43, the lysine residue at amino acid position 55, the lysine residue at amino acid position 95, the asparagine residue at amino acid position 137, the glutamine residue at amino acid position 138, the lysine residue at amino acid position 139, the arginine residue at amino acid position 144, the lysine residue at amino acid position 147, the glutamine residue at amino acid position 152, the lysine residue at amino acid position 153, or the threonine residue at amino acid position 154 of the sequence depicted in claim 1.

3. The polypeptide analog according to claim 1 selected from R(144)Q; C(1,15)S/R(144)E; C(1,15)S/R(144)Q; ΔN23/R(144)Q; ΔN23/N(137)E; ΔN23/K(139)E; ΔN23/K(139)Q; ΔN23/R(144)A; ΔN23/R(144)E; ΔN23/R(144)L; ΔN23/K(147)E; ΔN23/K(147)Q; ΔN23/K(153)E; ΔN23/K(153)Q; or ΔN23/Q(152)E/K(153)E.

4. The polypeptide analog according to any one of claims 1 to 3 wherein said polypeptide analog is covalently attached to a chemical moiety.

5. The polypeptide analog according to claim 4 wherein said chemical moiety is polyethylene glycol.

6. The polypeptide analog according to any one of claims 1 to 5, wherein said polypeptide analog is lyophilized.

7. A pharmaceutical formulation comprising a therapeutically effective amount of a polypeptide analog according to any one of claims 1 to 6 and a pharmaceutically acceptable carrier.

8. A recombinant nucleic acid molecule encoding a polypeptide analog according to any one of claims 1 to 3.

9. A biologically functional plasmid or viral vector comprising a recombinant nucleic acid molecule according to claim 8.

10. A procaryotic or eucaryotic host cell containing a recombinant nucleic acid according to claim 8 or a biologically functional vector according to claim 9.

11. A procaryotic host cell according to claim 10 that is *E. coli*.

12. A eucaryotic host cell according to claim 10 that is a mammalian cell, preferably a Chinese hamster ovary cell.

13. A process for the production of a polypeptide analog according to any one of claims 1 to 6, the process comprising growth under suitable nutrient conditions a procaryotic or eucaryotic host cell according to any one of claims 10 to 12, in a manner allowing expression of the encoded polypeptide analog, and isolating the polypeptide analog so produced.

14. An in vitro method of stimulating the production of non-fibroblast epithelial cells comprising contacting such cells with an effective amount of a polypeptide analog according to any one of claims 1 to 6 or a pharmaceutical formulation according to claim 7.

15. Use of a polypeptide analog according to any one of claims 1 to 6 for the preparation of a pharmaceutical formulation for the treatment of diseases requiring the stimulation of the production of non-fibroblast epithelial cells.

16. A use of an effective amount of a polypeptide analog according to any one of claims 1 to 6 for the production of a medicament for stimulation of non-fibroblast epithelial cells in a patient in need thereof.

17. The use according to claim 16, wherein said non-fibroblast epithelial cells are selected from adnexal structures, liver cells, mucosal epithelium in the respiratory and gastrointestinal tracts, comeal cells or tympanic epithelial cells.

18. A use of an effective amount of the polypeptide analog according to any one of claims 1 to 6 for the production of a medicament for stimulating production of non-fibroblast epithelial cells in a patient for the prevention or treatment of a condition, wherein said condition is selected from burns and other partial and full-thickness injuries; epidermolysis bullosa; chemotherapy-induced alopecia; male-pattern baldness; progressive loss of hair in men and women; gastric and duodenal ulcers; inflammatory bowel diseases such as Crohn's disease and ulcerative colitis; gut toxicity in radiation and chemotherapy treatment regimes; hyaline membrane disease; acute or chronic lung damage; hepatic cirrhosis, fulminant liver failure, acute viral hepatitis and/or toxic insults to the liver; corneal abrasion; progressive gum disease or ear drum damage.

19. A kit comprising a polypeptide analog according to any one of claims 1 to 6, or a pharmaceutical formulation of claim 7.

## Patentansprüche

1. Ein Polypeptidanalogon des als "KGF' bezeichneten nativen Keratinozytenwachstumsfaktors, wobei der native KGF mit der folgenden Sequenz korrespondiert: 
Cys
1

Asn Asp Met Thr Pro Glu Gln Met Ala Thr Asn Val Asn Cys Ser Ser
            5                   10                  15

Pro Glu Arg His Thr Arg Ser Tyr Asp Tyr Met Glu Gly Gly Asp Ile
        20                  25                  30

Arg Val Arg Arg Leu Phe Cys Arg Thr Gln Trp Tyr Leu Arg Ile Asp
    35                  40                  45

Lys Arg Gly Lys Val Lys Gly Thr Gln Glu Met Lys Asn Asn Tyr Asn
50 50 60 65

Ile Met Glu Ile Arg Thr Val Ala Val Gly Ile Val Ala Ile Lys Gly
                70                  75                  80

Val Glu Ser Glu Phe Tyr Leu Ala Met Asn Lys Glu Gly Lys Leu Tyr
            85                  90                  95

Ala Lys Lys Glu Cys Asn Glu Asp Cys Asn Phe Lys Glu Leu Ile Leu
        100                 105                 110

Glu Asn His Tyr Asn Thr Tyr Ala Ser Ala Lys Trp Thr His Asn Gly
    115                 120                 125

Gly Glu Met Phe Val Ala Leu Asn Gln Lys Gly Ile Pro Val Arg Gly
130                 135                 140                 145

Lys Lys Thr Lys Lys Glu Gln Lys Thr Ala His Phe Leu Pro Met Ala
                150                 155                 160

Ile Thr
 wobei besagtes Analogon eine erhöhte Stabilität aufweist, wobei das Analogon die Aminosäuresequenz von KGF umfasst, die einen Ladungswechsel durch die Deletion und/oder Substitution von einem oder mehreren der Aminosäurereste 41 - 154 der oben angegebenen Sequenz aufweist, wodurch eine verringerte positive Ladung im Vergleich zu nativem KGF bewirkt wird, wobei dieses optional zusätzlich eine Deletion der ersten 15 bis 24 Aminosäuren des N-terminalen nativen KGF, der zu Cys (1) und Cys (15) korrespondierende ersetzte oder deletierte Reste aufweist, ein N-terminales Methionin aufweist oder eine Signalsequenz aufweist, mit der Proviso, dass die Aminosäuresequenz 123-131 der oben angegebenen Sequenz nicht durch eine der Aminosäuresequenzen, ausgewählt aus der Gruppe DLYQG und AKYEG, substituiert ist.

2. Das Polypeptidanalogon gemäß Anspruch 1, wobei die deletierten oder substituierten Aminosäurereste ausgewählt sind aus dem Argininenrest in der Aminosäureposition 41, dem Glutaminrest in der Aminosäureposition 43, dem Lysinrest in der Aminosäureposition 55, dem Lysinrest in der Aminosäureposition 95, dem Asparaginrest in der Aminosäureposition 137, dem Glutaminrest in der Aminosäureposition 138, dem Lysinrest in der Aminosäureposition 139, dem Argininrest in der Aminosäureposition 144, dem Lysinrest in der Aminosäureposition 147, dem Glutaminrest in der Aminosäureposition 152, dem Lysinrest in der Aminosäureposition 153 oder dem Threoninrest in der Aminosäureposition 154 der in Anspruch 1 gezeigten Sequenz.

3. Das Polypeptidanalogon gemäß Anspruch 1, ausgewählt aus R(144)Q; C(1,15)S/R(144)E; C(1,5)S/R(144)Q; ΔN23/R(144)Q; ΔN23/N(137)E; ΔN23/K(139)E; ΔN23/K(139)Q; ΔN23/R(144)A; ΔN23/R(144)E; ΔN23/R(144)L; ΔN23/K(147)E; ΔN23/K(147)Q; ΔN23/K(153)E; ΔN23/K(153)Q; oder ΔN23/Q(152)E/K(153)E.

4. Das Polypeptidanalogon gemäß einem der Ansprüche 1 bis 3, wobei besagtes Polypeptidanalogon kovalent an einen chemischen Bereich gebunden ist.

5. Das Polypeptidanalogon gemäß Anspruch 4, wobei besagter chemischer Bereich Polyethylenglycol ist.

6. Das Polypeptidanalogon gemäß einem der Ansprüche 1 bis 5, wobei besagtes Polypeptidanalogon lyophilisiert ist.

7. Eine pharmazeutische Formulierung, umfassend eine therapeutisch wirksame Menge eines Polypeptidanalogon gemäß einem der Ansprüche 1 bis 6 und einen pharmazeutisch verträglicher Träger.

8. Ein rekombinantes Nukleinsäuremolekül, dass ein Polypeptidanalogon gemäß einem der Ansprüche 1 bis 3 kodiert.

9. Ein biologisch funktionelles Plasmid oder viraler Vector, umfassend ein rekombinantes Nukleinsäuremolekül gemäß Anspruch 8.

10. Eine prokariontische oder eukariontische Wirtszelle, enthaltend eine rekombinante Nukleinsäure gemäß Anspruch 8 oder einen biologisch funktionalen Vector gemäß Anspruch 9.

11. Eine prokariontische Wirtszelle gemäß Anspruch 10, die *E. coli* ist.

12. Eine eukariontische Wirtszelle gemäß Anspruch 10, die eine Säugetierzelle ist, vorzugsweise eine chinesische Hamsterovarzelle.

13. Ein Verfahren für die Herstellung eines Polypeptidanalogons gemäß einem der Ansprüche 1 bis 6, wobei das Verfahren das Wachsen einer prokariontischen oder eukariontischen Wirtszelle gemäß einem der Ansprüche 10 bis 12 unter geeigneten Nährstoffbedingungen in einer Weise umfasst, die die Expression des kodierten Polypeptidanalogons zulässt und die Isolierung des so hergestellten Polypeptidanalogons.

14. Ein *in vitro*-Verfahren zur Stimulation der Produktion von Nichtfibroblasten-Epithelzellen, umfassend das in Kontaktbringen solcher Zellen mit einer wirksamen Menge eines Peptidanalogons gemäß einem der Ansprüche 1 bis 6 oder einer pharmazeutischen Formulierung gemäß Anspruch 7.

15. Verwendung eines Polypeptidanalogons gemäß einem der Ansprüche 1 bis 6 für die Herstellung einer pharmazeutischen Formulierung für die Behandlung von Krankheiten, die die Stimulation der Produktion von Nichtfibroblasten-Epithelzellen benötigen.

16. Eine Verwendung einer wirksamen Menge eines Polypeptidanalogons gemäß einem der Ansprüche 1 bis 6 für die Herstellung eines Medikaments zur Stimulation von Nichtfibroblasten-Epithelzellen in einem Patienten, der dessen bedarf.

17. Die Verwendung gemäß Anspruch 16, wobei besagte Nichtfibroblasten-Epithelzellen ausgewählt sind aus adnexalen Strukturen, Leberzellen, Mukosaepithel in den respiratorischen und gastrointestinalen Trakten, komealen Zellen oder tympanische Epithelzellen.

18. Eine Verwendung einer wirksamen Menge des Polypeptidsanalogons gemäß einem der Ansprüche 1 bis 6 für die Herstellung eines Medikaments zur Stimulation der Produktion vom Nichtfibroblasten-Epithelzellen in einem Patienten für die Prävention oder Behandlung eines Zustandes, wobei besagter Zustand ausgewählt ist aus Verbrennungen oder anderen Verletzungen teilweiser oder vollständiger Dicke; Epidermolyse bullosa; Chemotherapie-indizierter Alopecia; männliche Musterglatzköpfigkeit; progressiver Verlust von Haar in Männern und Frauen; Magen- und Zwölffingerdarmgeschwüre; entzündliche Darmkrankheiten wie Crohn's-Krankheit und geschwürartiger Darmkatarrh; Darmtoxizität bei Strahlungs- und Chemotherapiebehandlungen; Hyaline Membrankrankheit; akuter oder chronischer Lungenschaden; Leberzirrhose, fulminantes Leberversagen, akute virale Hepatitis und/oder toxische Angriffe auf die Leber; komeale Abschürfungen; progressive Zahnfleischerkrankung oder Trommelfellschaden.

19. Ein Kit, umfassend ein Polypeptidanalogon gemäß einem der Ansprüche 1 bis 6 oder eine pharmazeutische Formulierung von Anspruch 7.

## Revendications

1. Analogue polypeptidique du facteur de croissance kératinocytaire ("KGF") natif, dans lequel le KGF natif correspond à la séquence suivante 
Cys
1

Asn Asp Met Thr Pro Glu Gln Met Ala Thr Asn Val Asn Cys Ser Ser
            5                   10                  15

Pro Glu Arg His Thr Arg Ser Tyr Asp Tyr Met Glu Gly Gly Asp Ile
        20                  25                  30

Arg Val Arg Arg Leu Phe Cys Arg Thr Gln Trp Tyr Leu Arg Ile Asp
    35                  40                  45

Lys Arg Gly Lys Val Lys Gly Thr Gln Glu Met Lys Asn Asn Tyr Asn
50                  55                  60                  65

Ile Met Glu Ile Arg Thr Val Ala Val Gly Ile Val Ala Ile Lys Gly
                70                  75                  80

Val Glu Ser Glu Phe Tyr Leu Ala Met Asn Lys Glu Gly Lys Leu Tyr
            85                  90                  95

Ala Lys Lys Glu Cys Asn Glu Asp Cys Asn Phe Lys Glu Leu Ile Leu
        100                 105                 110

Glu Asn His Tyr Asn Thr Tyr Ala Ser Ala Lys Trp Thr His Asn Gly
    115                 120                 125

Gly Glu Met Phe Val Ala Leu Asn Gln Lys Gly Ile Pro Val Arg Gly
130                 135                 140                 145

Lys Lys Thr Lys Lys Glu Gln Lys Thr Ala His Phe Leu Pro Met Ala
                150                 155                 160

Ile Thr
 ledit analogue ayant une stabilité améliorée, dans lequel l'analogue comporte la séquence peptidique du KGF ayant une modification de charge par délétion et/ou substitution d'un ou de plusieurs résidus acides aminés 41-154 de la séquence ci-dessus pour aboutir à une réduction de la charge positive par rapport au KGF natif, ledit KGF natif, ayant en outre, facultativement, une délétion des 15 à 24 premiers acides aminés de l'extrémité N-terminale du KGF natif, les résidus Cys(1) et Cys(15) étant remplacés ou délétés, et ayant une séquence signal ou une méthionine en position N-terminale, avec cette réserve que la séquence peptidique 123-131 de la séquence ci-dessus n'est substituée par aucune des séquences peptidiques sélectionnées à partir des groupes DLYQG et AKYEG.

2. Analogue polypeptidique selon la revendication 1, dans lequel les résidus acides aminés délétés ou substitués sont sélectionnés à partir du résidu arginine en position 41, du résidu glutamine en position 43, du résidu lysine en position 55, du résidu lysine en position 95, du résidu asparagine en position 137, du résidu glutamine en position 138, du résidu lysine en position 139, du résidu arginine en position 144, du résidu lysine en position 147, du résidu glutamine en position 152, du résidu lysine en position 153 ou du résidu thréonine en position 154 de la séquence décrite dans la revendication 1.

3. Analogue polypeptidique selon la revendication 1, sélectionné à partir de R (144)Q ; C(1,15)S/R(144)E ; C(1,5)S/R(144)Q ; ΔN23/R(144)Q ; ΔN23/N(137)E ; ΔN23/K(139)E ; ΔN23/K(139)Q ; ΔN23/R(144)A ; ΔN23/R(144)E ; ΔN23/R(144)L ; ΔN23/K(147)E ; ΔN23/K(147)Q ; ΔN23/K(153)E; ΔN23/K(153)Q ; ou ΔN23/Q(152)E/K(153)E.

4. Analogue polypeptidique selon l'une quelconque des revendications 1 à 3, dans lequel ledit analogue polypeptique est lié de façon covalente à une fraction chimique.

5. Analogue polypeptidique selon la revendication 4, dans lequel ladite fraction chimique est le polyéthylène glycol.

6. Analogue polypeptidique selon l'une quelconque des revendications 1 à 5, dans lequel ledit analogue polypeptidique est lyophilisé.

7. Formulation pharmaceutique comportant une quantité thérapeutiquement active d'un analogue polypeptidique salon l'une quelconque des revendications 1 à 6 et un transporteur pharmaceutiquement acceptable.

8. Molécule d'acide nucléique recombinée codant pour un analogue polypeptidique selon l'une quelconque des revendications 1 à 3.

9. Plasmide ou vecteur viral biologiquement fonctionnel comportant une molécule d'acide nucléique recombinée selon la revendication 8.

10. Cellule-hôte, procaryote ou eucaryote, contenant un acide nucléique recombiné selon la revendication 8 ou un vecteur biologiquement fonctionnel selon la revendication 9.

11. Cellule-hôte procaryote selon la revendication 10 qui *est E. coli.*

12. Cellule-hôte eucaryote selon la revendication 10 qui est une cellule de mammifère, de préférence une cellule d'ovaire de hamster chinois.

13. Procédé pour la production d'un analogue polypeptidique selon l'une quelconque des revendications 1 à 6, le procédé comportant une croissance, dans des conditions de nutriments appropriées, dans une cellule procaryote ou eucaryote, selon l'une quelconque des revendications 10 à 12, d'une manière permettant l'expression de l'analogue polypeptidique codé, et un isolement de l'analogue polypeptidique ainsi produit.

14. Méthode in vitro de stimulation de la production de cellules épithéliales non fibroblastiques consistant à mettre en contact ces cellules avec une quantité efficace d'un analogue polypeptidique selon l'une quelconque des revendications 1 à 6 ou d'une formulation pharmaceutique selon la revendication 7.

15. Utilisation d'un analogue polypeptidique selon l'une quelconque des revendications 1 à 6 pour la préparation d'une formulation pharmaceutique pour le traitement d'affections nécessitant de stimuler la production de cellules épithéliales non fibroblastiques.

16. Utilisation d'une quantité efficace d'un analogue polypeptidique selon l'une quelconque des revendications 1 à 6 pour la production d'un médicament pour stimuler la production de cellules épithéliales non fibroblastiques chez un patient qui le nécessite.

17. Utilisation selon la revendication 16, dans lequel lesdites cellules épithéliales non fibroblastiques sont sélectionnées à partir de structures annexielles, d'hépatocytes, d'épithélium muqueux des tractus respiratoire et digestif, de cellules de la cornée ou du tympan.

18. Utilisation d'une quantité efficace de l'analogue plypeptidique selon l'une quelconque des revendications 1 à 6 pour la production d'un médicament pour stimuler la production de cellules épithéliales non fibroblastiques chez un patient pour la prévention ou le traitement d'une affection, ladite affection entrant parmi les pathologies suivantes : brûlures et autres lésions cutanées superficielles ou profondes ; épidermolyse bulleuse ; alopécie induite par la chimiothérapie ; calvitie androgénique ; chute progressive des cheveux chez les deux sexes ; ulcères gastriques et duodénaux ; colopathies inflammatoires de type maladie de Crohn et rectocolite hémorragique ; toxicité intestinale de la radiothérapie et de la chimiothérapie ; maladie des membranes hyalines ; pneumopathies aiguës ou chroniques ; cirrhose hépatique ; hépatite fulminante ; hépatite virale aiguë ; hépatites toxiques ; abrasion cornéenne ; gingivite évolutive ou lésions du tympan.

19. Trousse contenant un analogue polypeptidique selon l'une quelconque des revendications là 6 ou une formulation pharmaceutique selon la revendication 7.
